# EUROPEAN PATENT APPLICATION

(11) **EP 4 555 961 A1**
(43) Date of publication of application: **21.05.2025**
(21) Application number: 23839999.2
(22) Date of filing: 13.07.2023
(51) Int. Cl.: A61B 34/30, A61B 17/22, A61B 1/00, A61B 34/37, A61B 90/00, A61B 17/00

(54) **SURGICAL ROBOT HAVING FORCE SENSING UNIT**

(30) Priority: 13.07.2022 KR 20220086570; 13.07.2022 KR 20220086610; 07.07.2023 KR 20230088668
(71) Applicant: ROEN Surgical, Inc., Daejeon 34051 (KR)
(72) Inventor: YANG, Un Je, Daejeon 34051 (KR); LEE, Dong Ho, Daejeon 34051 (KR); KIM, Joon Yeong, Daejeon 34051 (KR); KIM, Joon Hwan, Daejeon 34051 (KR); SONG, Kyu Seob, Daejeon 34051 (KR); KONG, Duk Yoo, Daejeon 34051 (KR); TUANI, De Sa Rosa, Daejeon 34051 (KR)
(74) Representative: Stolmár & Partner Patentanwälte PartG mbB
(86) International application number: PCT/KR2023/010056
(87) International publication number: WO 2024/014908

(57) **Abstract**

The present invention may provide a surgical robot comprising: an endoscope having an overtube that is inserted into the human body; a surgical tool inserted into the overtube; and a force sensing unit which measures the force that is generated when inserting or withdrawing the endoscope into or from the human body.

## Description

### Technical Field

The present disclosure relates to a surgical robot having a force sensing unit. More particularly, the present disclosure relates to a surgical robot having a force sensing unit, the surgical robot being configured such that a force that may be generated during a surgical operation is capable of being measured through the force sensing unit, thereby enabling the surgical operation to be performed safely.

### Background Art

Generally, a stone existing in the human body is formed by deposition of organic ions due to the change in solubility according to the change in pH. The stone may be classified into a ureteric stone, a bile duct stone (gallstone) and so on according to an area where the stone is formed. When such a stone is formed, the stone causes flow disorder and causes symptoms accordingly.

There are various methods for removing such a stone, and such a stone is usually removed by using an endoscope, a basket catheter, an electrohydraulic lithotripter having a lithotripsy probe, and so on.

In order to remove a kidney stone, an access sheath is inserted into the ureter of the human body, and an overtube is moved inside of the access sheath, so that the stone is removed. The access sheath serves to protect an inner wall of the ureter so that the overtube does not damage the inner wall of the ureter.

However, in order to remove the stone, the access sheath receives a force by being in contact with the ureter during a process of positioning the access sheath in the ureter, so that the inner wall of the ureter may be damaged by the access sheath.

In addition, when a process of inserting the overtube into the access sheath or a process of removing a broken stone is performed in order to remove the stone, the overtube may be stuck on an end portion of the access sheath due to the stone and the overtube may not be smoothly withdrawn from the access sheath. At this time, the access sheath receives a force caused by a forward or backward movement of the overtube, and the access sheath may fluctuate, so that the access sheath may damage to the inner wall of the ureter.

In addition, in a situation in which a stone having a diameter larger than a diameter (an inner diameter) of the access sheath is attempted to be pulled through the access sheath, when an attempt is forcibly made to pull the stone even though the stone is caught on a distal end portion of the access sheath, the inner wall of the ureter may be damaged, such as tearing of the ureter and so on.

In addition, in a process of performing a surgical operation through the endoscope after the access sheath is inserted into the ureter, the ureter may be moved by a patient's breath. In this situation, a pushing force by the moving ureter may be applied to the access sheath that is in a fixed state, and it is necessary to respond such a force by the ureter applied to the access sheath so that the ureter does not adversely affect the ureter.

When a stone is removed, the access sheath may move and may damage to the inner wall of the ureter due to friction or jam of the endoscope or the stone on the access sheath, so that it is necessary to prevent damage to the inner wall of the ureter by measuring a force acting on the access sheath due to various causes and to perform the surgical operation safely.

In addition, when the access sheath is not inserted into the ureter but the endoscope is inserted directly into the ureter, friction or jam may occur on the inner wall of the ureter due to a movement of the endoscope and/or a surgical tool provided in the overtube of the endoscope. Therefore, it is necessary to prevent damage to the inner wall of the ureter during performing the surgical operation by measuring a force acting on the endoscope or the surgical tool.

### Disclosure

### Technical Problem

An objective of the present disclosure is to provide a surgical robot being configured such that a force acting on an access sheath or an endoscope inserted into the human body is measured during a surgical operation using the endoscope, the surgical robot being configured to perform a safety action when the force is equal to or more than a reference value, thereby being capable of increasing the safety of the surgical operation.

### Technical Solution

According to the present disclosure, there is provided a surgical robot including: an endoscope having an overtube that is configured to be inserted into an inner portion of the human body; a surgical tool configured to be inserted into the inner portion of the human body; and a force sensing unit configured to measure a force generated when the endoscope is inserted into or withdrawn from the inner portion of the human body.

According to the present disclosure, there is provided a surgical robot including: an endoscope having an overtube that is configured to be inserted into an inner portion of the human body; a surgical tool configured to be inserted into the inner portion of the human body; an access sheath configured to be inserted into the inner portion of the human body; and a force sensing unit configured to measure a force acting between the human body and the access sheath.

### Advantageous Effects

As such, in a surgical operation for removing a kidney stone of a patient for example, when the overtube of the endoscope is stuck inside the access sheath or friction is increased during a process of inserting the access sheath for protecting the ureter into the inner portion of the ureter and a process of inserting the endoscope for removing the stone into the inner portion of the access sheath, a pushing force acting on the access sheath is capable of being measured by the force sensing unit. Furthermore, when the force exceeds a reference value, the operation of the surgical robot may be stopped, or an alarm may be sounded to notify a user.

In addition, when the stone is stuck on the access sheath during a process of passing through the access sheath for removing the stone from outside the patient's body by using the endoscope, a pulling force that moves the access sheath backward is applied, and the pushing force and the pulling force applied to the access sheath are measured by the force sensing unit. Furthermore, when the force exceeds the reference value, the operation of the surgical robot may be stopped, or an alarm may be sounded and a subsequent action may be performed.

In addition, in the present disclosure, the force acting on the access sheath is capable of being measured by the force sensing unit. Therefore, when the measured value exceeds the reference value, a setting position of the access sheath may be changed so as to remove the excess force, and the surgical robot may be reused.

The surgical robot according to the present disclosure includes a mounting unit on which the endoscope is mounted and which is configured to be reciprocated, a moving unit configured to allow the mounting unit to reciprocate, and a holder provided on the moving unit and configured to hold the access sheath. Furthermore, the holder includes a holding part configured to hold the access sheath, and includes an arm part connected to the holding part and configured to be rotated by being connected to an inner portion of the moving unit. Furthermore, the force sensing unit configured to measure a force acting when the arm part is rotated is provided on an end side of the arm part, so that the force of both directions applied to the access sheath is capable of being immediately measured.

Therefore, by the force sensing unit, the present disclosure may serve as a safety device in the process of performing the surgical operation.

### Description of Drawings

FIG. 1 is a front view illustrating a surgical robot according to an embodiment of the present disclosure.
FIG. 2 is a perspective view illustrating a moving unit and a mounting unit that are forming the surgical robot according to an embodiment of the present disclosure.
FIG. 3 is a perspective view illustrating an internal structure of the moving unit in the surgical robot according to an embodiment of the present disclosure.
FIG. 4 is an explanatory view for describing a principle in which a force is measured by a force sensing unit in the surgical robot according to an embodiment of the present disclosure.

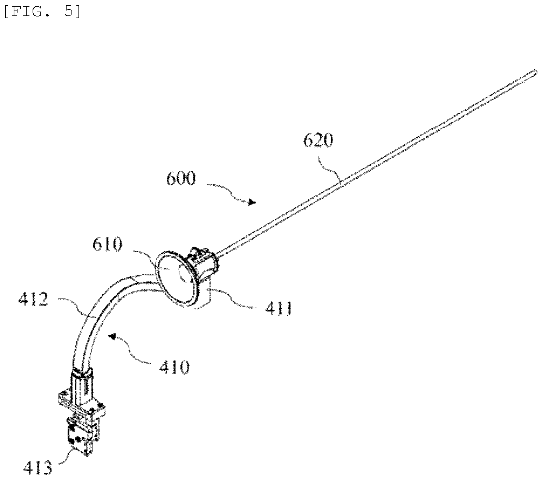

FIG. 5 is a perspective view illustrating a state in which a support holder and an access sheath of the present disclosure are coupled to each other.

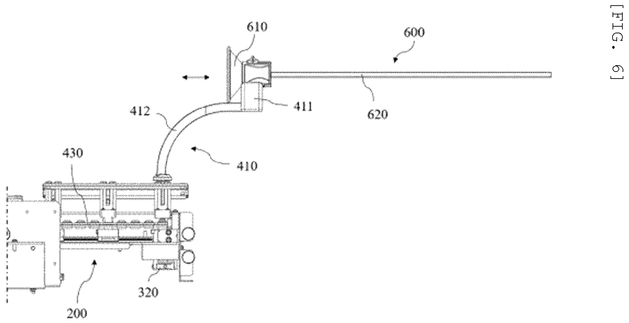

FIG. 6 is a view illustrating an arrangement relationship between the force sensing unit and the support holder on which the access sheath of the surgical robot according to an embodiment of the present disclosure is held.
FIG. 7 is a cross-sectional view illustrating a state in which the access sheath and an endoscope of the surgical robot according to an embodiment of the present disclosure are inserted into the kidney and a stone is removed.
FIG. 8 is a cross-sectional view illustrating a state in which the stone is held by the endoscope inserted into the access sheath of the surgical robot according to an embodiment of the present disclosure.

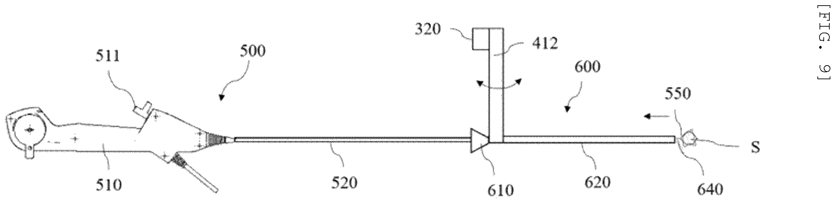

FIG. 9 is a schematic view illustrating a state in which a force is measured by the force sensing unit when the force is applied to the access sheath while the endoscope according to an embodiment of the present disclosure penetrates an inner portion of the access sheath and removes the stone.
FIG. 10 is a view illustrating a configuration in which a force applied to the access sheath according to an embodiment of the present disclosure is measured, a control unit determines the force, and then a result of determination is notified through an alarm unit when the force is equal to or more than a reference value.
FIG. 11 is a view illustrating a surgical robot according to an embodiment.
FIG. 12 is a perspective view illustrating a driving part of a slave device according to an embodiment.
FIG. 13 is a perspective view illustrating an endoscopic device, a coupler, and a mount manufactured by Boston Scientific according to an embodiment.
FIG. 14 is a perspective view illustrating the coupler according to an embodiment.
FIG. 15 is a perspective view illustrating a coupler supporting an endoscopic device manufactured by Karl Storz according to an embodiment.
FIG. 16 is a perspective view illustrating a coupler supporting an endoscopic device manufactured by OTU Medical according to an embodiment.

### Mode for Invention

Hereinafter, embodiments of the present disclosure will be described in detail with reference to the accompanying drawings. However, the technical spirit of the present disclosure is not limited to some of the described embodiments, but may be implemented in various different forms, and as long as it is within the scope of the technical spirit of the present disclosure, one or more of the components may be selectively combined and/or substituted between the embodiments.

In addition, terms used in the embodiments of the present disclosure may be generally understood by those of ordinary skilled in the art to which the present disclosure belongs, unless specifically defined and described explicitly, and terms commonly used, such as terms defined in the dictionary, may be interpreted in consideration of the contextual meaning of the related art.

In addition, the terms of the embodiments of the present disclosure are for description of the embodiments and do not limit the present disclosure, and singular forms may be interpreted as including plural forms unless the context clearly indicates otherwise.

In addition, in the components of the embodiments of the present disclosure, terms such as first, second, and third, or A, B, and C, and so on may be used, and these terms are only for distinguishing one component from other components and does not limit order or sequence thereof.

In addition, in the embodiment of the present disclosure, when one component is described as "connected", "bonded", "coupled", and so on to another component, it may mean that the one component is directly connected, bonded, or coupled to the another component, and two components are indirectly connected, bonded, or coupled to each other by another component therebetween.

In addition, in the embodiment of the present disclosure, the placement, formation, and positioning of one component on, beneath, above, or under another component may mean that the one component is directly or indirectly placed, formed, or positioned on, above, or under the another component. Expressions such as up or down, and an upper or lower side may mean not only an upward direction but also a downward direction with respect to one component.

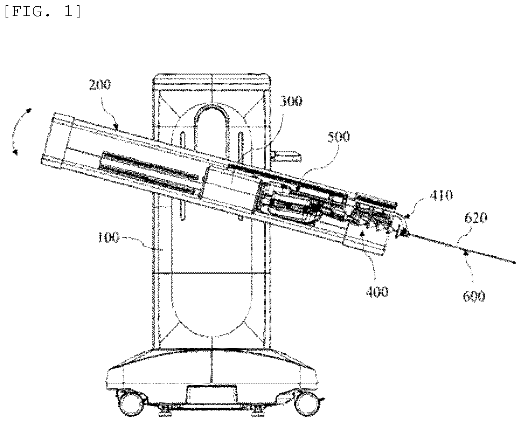

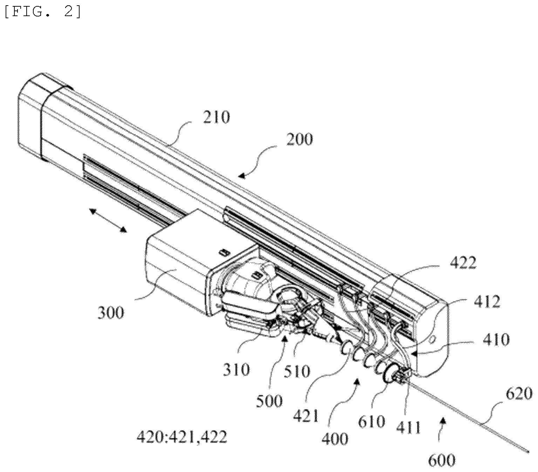

FIG. 1 is a front view illustrating an embodiment of a surgical robot of the present disclosure, and FIG. 2 is a perspective view illustrating a moving unit and a mounting unit forming the surgical robot according to an embodiment of the present disclosure.

Referring to FIG. 1 and FIG. 2, a surgical robot according to an embodiment of the present disclosure may include a main body unit 100, a moving unit 200 provided on the main body unit 100, a mounting unit 300 which is provided on a first surface of the moving unit 200 such that the mounting unit 300 is capable of reciprocating and sliding on the first surface of the moving unit 200 and which is provided such that an endoscope 500 is capable of being attached to and detached from the mounting unit 300, and a holding unit 400 configured to hold and move the endoscope 500 and an access sheath 600.

A controller and a control panel that are capable of controlling an overall operation of the surgical robot may be provided on the main body unit 100.

The moving unit 200 may include a casing 210 having a predetermined length, and may be coupled to the main body unit 100 such that the moving unit 200 is capable of being rotated with respect to the main body unit 100 by a driving mechanism (a motor and so on). Furthermore, the mounting unit 300 may be provided on a first surface of the casing 210 such that the mounting unit 300 is capable of rectilinearly reciprocating along a length direction of the first surface of the casing 210 by a driving mechanism (a motor, a rectilinear movement apparatus, and so on).

A mounting part 310 capable of mounting the endoscope 500 thereon may be provided on the mounting unit 300.

The mounting part 310 may be coupled to a body part of the mounting unit 300 such that the mounting part 310 is capable of being rotated. Therefore, the mounting part 310 may be connected to a driving mechanism (a motor and so on) provided in the body part of the mounting unit 300. Accordingly, the mounting part 310 may be configured to be rotated in a forward direction and a reverse direction by a rotation in a forward direction and a reverse direction of the driving mechanism, and the endoscope 500 may be rotated in a forward direction and a reverse direction accordingly.

The holding unit 400 may include a support holder 410 capable of holding the access sheath 600, and may include and a guide holder 420 which is provided as a plurality of guide holders 420 spaced apart from each other by a predetermined distance and which is capable of stably guiding the endoscope 500.

The guide holder 420 may include an insertion member 421 formed in a cone shape through which an overtube 520 of the endoscope 500 penetrates, and may include a connection member 422 which extends from the insertion member 421 and which is connected to the casing 210 of the moving unit 200.

The guide holder 420 may support the overtube 520 of the endoscope 500, and may prevent buckling of the endoscope 500 when the mounting unit 300 is moved with respect to the moving unit 200 or when the mounting part 310 is rotated.

In addition, the holding unit 400 may be provided with a folding member 430 which supports the plurality of guide holders 420 and which is configured to be moved such that the distance between the plurality of guide holders 420 is capable of being adjusted and also the adjusted distance is maintained.

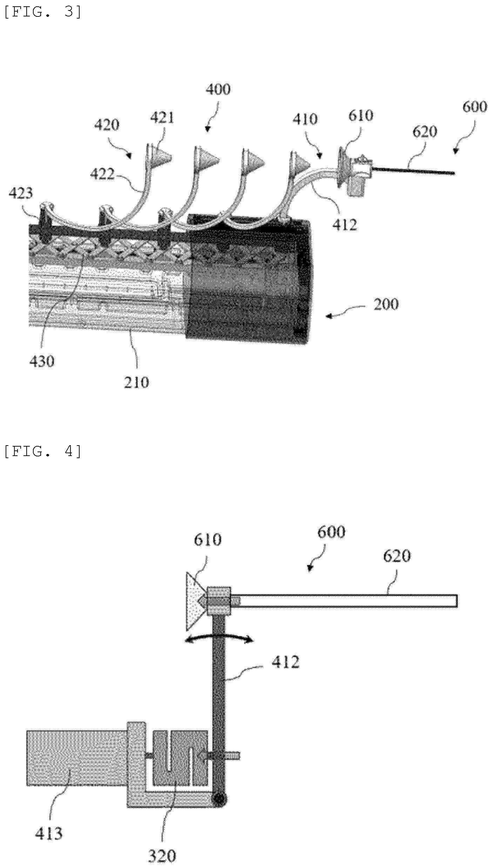

Referring to FIG. 3, the plurality of guide holders 420 may be coupled to the folding member 430 by a coupling member 423 while being spaced at each point where two X-shaped links intersect in the folding member 430.

For example, the folding member 430 may be formed of a plurality of links which has X shapes and which is connected to each other such that a length thereof is capable of being changed.

Therefore, by an adjusted length of the folding member 430, the distance between the plurality of support holders 410 may be maintained at the equal distance.

The endoscope 500 may include an operation member 510, and may include the overtube 520 which extends by a predetermined length from the operation member 510 and which is configured to be operated by the operation member 510. A surgical tool such as a laser part having a wire (an optical fiber) shape or a basket for removing a stone may be inserted into an inner portion of the overtube 520. Therefore, the overtube 520 of the endoscope 500 may penetrate an inner portion of a protective tube 620.

When the endoscope 500 is mounted on the mounting part 310 of the mounting unit 300, the overtube 520 penetrates the insertion member 421 of the guide holder 420, and may be inserted into an inner portion of the access sheath 600.

The operation member 510 may be provided with an insertion tube 511 capable of inserting a laser part or a basket connected by a wire into the inner portion of the overtube 520.

The operation member 510 may be provided on the mounting part 310 of the mounting unit 300 such that the operation member 510 is capable of being attached to and detached from the mounting part 310 of the mounting unit 300. The overtube 520 having the predetermined length is provided at a tip end of the operation member 510. Therefore, when the operation member 510 is mounted on the mounting part 310 of the mounting unit 300, the overtube 520 may penetrate the guide holder 420 along a transverse direction and may be inserted into the inner portion of the access sheath 600.

For example, when a surgical operation for removing a stone in the kidney, the access sheath 600 may perform a protecting function to prevent damage to the inner wall of the ureter U when a process of repeatedly inserting or withdrawing the endoscope 500 inside the ureter U.

The access sheath 600 may include an inlet member 610 which has a cone shape and which is held on the support holder 410, and may include the protective tube 620 that extends by a predetermined length from the inlet member 610

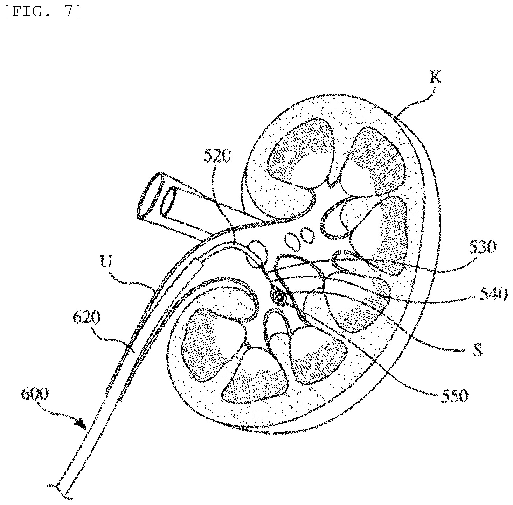

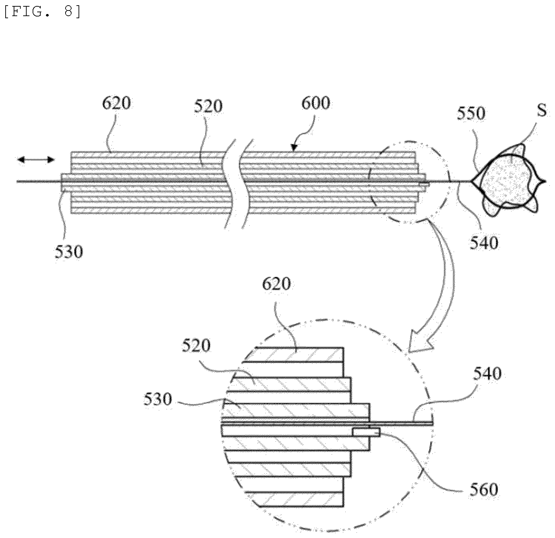

Referring to FIG. 7 and FIG. 8, in order to remove a stone S formed in the kidney K, the access sheath 600 is inserted through the ureter U of a patient and the overtube 520 of the endoscope 500 is inserted into the inner portion of the access sheath 600. At this time, as a surgical tool, a guide tube 530, a wire 540 that penetrates an inner portion of the guide tube 530, a basket which is provided on an end portion of the wire 540 and which is capable of holding a stone so as to remove the stone may be included in the inner portion of the overtube 520.

The support holder 410 may include a holding part 411 on which the inlet member 610 of the access sheath 600 is held such that the inlet member 610 of the access sheath 600 is capable of being attached to and detached from the holding part 411, and may include an arm part 412 connected from the holding part 411 to the casing 210 of the moving unit 200.

The holding part 411 has an elasticity, and may have an opening part so that a portion of the inlet member 610 of the access sheath 600 is capable of being attached to and detached from the opening part. Therefore, when the inlet member 610 of the access sheath 600 is inserted through the opening part of the holding part 411, the opening part expands, and the inlet member 610 may be inserted inside and mounted in the holding part 411.

A mounting position of the support holder 410 and a force sensing unit 320 on the moving unit 200 may be changed and fixed as required. This may be for setting the access sheath 600 while an appropriate distance is set so that a force is not applied to the force sensing unit 320 when initial setting is performed according to a distance between the surgical robot and the patient.

The support holder 410 and the force sensing unit 320 are connected to an actuator (a motor, a linear screw, and an LM guide) that is separately provided, so that the support holder 410 and the force sensing unit 320 are capable of being rectilinearly reciprocated.

Furthermore, when the access sheath 600 is inserted into the ureter U of the patient, it may be preferable that the access sheath 600 is inserted into the ureter U of the patient while the access sheath 600 is in a state in which an insertion position of the access sheath 600 is aligned. This is because, when the insertion position is not aligned, a force may occur as a result, and may affect the force sensing unit 320. That is, it is necessary to measure a pure force in a state in which an initial value is set to zero, the pure force being generated by an operation of the overtube 520 that is inserted into the access sheath 600.

In an embodiment of the present disclosure, the arm part 412 may extend from the casing 210 of the moving unit 200 and may be bent downwardly, and an end portion of the arm part 412 and the holding part 411 may be connected to each other.

Therefore, the cone-shaped insertion member 421 of the plurality of guide holders 420 and the holding part 411 may be disposed in a line, and the overtube 520 of the endoscope 500 may be easily inserted into the inner portion of the access sheath 600.

At this time, according to an embodiment of the present disclosure, the force sensing unit 320 capable of measuring a force according to a movement of the arm part 412 may be fixed inside the casing 210 of the moving unit 200. In other words, the force sensing unit 320 may be coupled to and fixed to a bracket 413 fixed inside the casing 210 of the moving unit 200, and may be in a state in which the force sensing unit 320 is capable of being in contact with an end portion of the arm part 412 of the support holder 410.

For example, the force sensing unit 320 may include various sensors capable of measuring an axial force, such as a load cell, a strain gauge, a Fiber Bragg Gratings (FBG) sensor, a piezo sensor, and so on.

The force sensing unit 320 may measure a force acting according to a forward movement and/or a backward movement of the endoscope 500 that is moved inside the access sheath 600.

In addition, the force sensing unit 320 may measure a force applied to the access sheath 600 due to friction or a jam of the endoscope 500 or a stone inside the access sheath 600 during a process of removing the stone through the endoscope 500 after the access sheath 600 is inserted into the ureter.

In addition, the force sensing unit 320 may measure a force acting on the access sheath 600 by the ureter during a process of inserting the access sheath 600 into the ureter and mounting the access sheath 600 on the support holder 410 in a state before the endoscope 500 is inserted.

In addition, the force sensing unit 320 may measure a force applied to the access sheath 600 according to a movement of the ureter by the patient's breath during a surgical operation in a state in which the access sheath 600 is mounted on the support holder 410.

When a force is applied to the access sheath 600 mounted on the support holder 410, the arm part 412 may be rotated with respect to the end portion of the arm part 412 or may be moved in a parallel manner, and such a force causing the movement of the arm part 412 may be measured in the force sensing unit 320.

Referring to FIG. 4, in a situation in which the end portion of the arm part 412 is connected and is acting as a hinge point, when a force is applied to the arm part 412, the arm part 412 may move while drawing an arc around the hinge point. However, the displacement is small, and the movement of the arm part 412 may be regarded as a rectilinear movement. Furthermore, when a force is applied to the force sensing unit 320 by such a hinge operation, the applied force may be measured. At this time, the force sensing unit 320 may be coupled to the bracket 413 fixed inside the casing 210 of the moving unit 200 and may maintain a fixed state.

Alternatively, the end portion of the arm part 412 may be provided such that the end portion of the arm part 412 is capable of being moved in the parallel manner to apply a force without applying a force to the force sensing unit 320 by the hinge operation. In such a situation, at least one guide bush capable of guiding the parallel movement may be provided on the end portion of the arm part 412.

Meanwhile, a surgical operation may be performed by inserting only the endoscope 500 into the ureter without inserting the access sheath 600 into the ureter that is an inner part of the human body.

In this situation, when the endoscope 500 is inserted and withdrawn, a force is applied by the endoscope 500 in contact with the ureter and so on. Therefore, it is necessary to measure the force according to the movement of the endoscope 500.

Therefore, in a state in which the access sheath 600 is not held on the support holder 410, the overtube 520 of the endoscope 500 may pass through the holding part 411 of the support holder 410 and may be inserted into the ureter where the surgical operation will be performed. At this time, since the overtube 520 of the endoscope 500 is mounted on the holding part 411 with a friction force, the support holder 410 may be moved by a force acting on the overtube 520 of the endoscope 500.

Accordingly, a force acting upon insertion or withdrawal into the ureter through the endoscope 500 may be transmitted to the arm part 412 of the support holder 410 through the holding part 411, and the force acting on the endoscope 500 may be measured by the force sensing unit 320.

Meanwhile, as another example, a force sensing unit may be provided on the mounting part 310 of the mounting unit 300 on which the operation member 510 of the endoscope 500 is mounted, and a force according to an axial (longitudinal direction) movement of the endoscope 500 may be measured. At this time, the mounting part 310 may serve as a holder holding the endoscope 500.

The force sensing unit provided on the mounting part 310 may be disposed in an axial direction in which the endoscope 500 receives a force.

When the mounting unit 300 is moved along the longitudinal direction from the moving unit 200, the operation member 510 of the endoscope 500 mounted on the mounting part 310 maintains a mounted state with a predetermined amount of friction force. Furthermore, when a movement according to a surgical operation of the endoscope 500 occurs, the operation member 510 is capable of reciprocating in the axial direction within the mounting part 310, and the force corresponding to the reciprocating movement may be measured by the force sensing unit provided on the mounting part 310.

In addition, a mounting position of the force sensing unit for measuring a force acting on the endoscope 500 is not limited thereto, and the force sensing unit may be provided between the mounting part 310 and the body part of the mounting unit 300 and may measure the force according to the axial (longitudinal direction) movement of the endoscope 500.

In other words, a force acting on the endoscope 500 may be a force acting upon insertion or withdrawal of the endoscope 500 into the ureter similar to a force acting on the access sheath 600, a force applied by the ureter due to the patient's breath during the surgical operation, a force generated in the ureter due to friction or jam during a stone removal operation after the surgical operation, and so on, and such a force may be measured by the force sensing unit.

The force sensing unit 320 is capable of measuring a force acting in both directions. Therefore, when forces (a pushing force and a pulling force) act in both directions of the support holder 410 or the endoscope 500, all the forces is capable of being measured by the force sensing unit 320.

In other words, the force sensing unit 320 may measure a force by being pressed or stretched as the pushing force and the pulling force are acting on the access sheath 600 or the endoscope 500. The end portion of the arm part 412 of the support holder 410 connected to the force sensing unit 320 or the endoscope 500 may be returned to the original position thereof by the force sensing unit 320 after the displacement by the application of force.

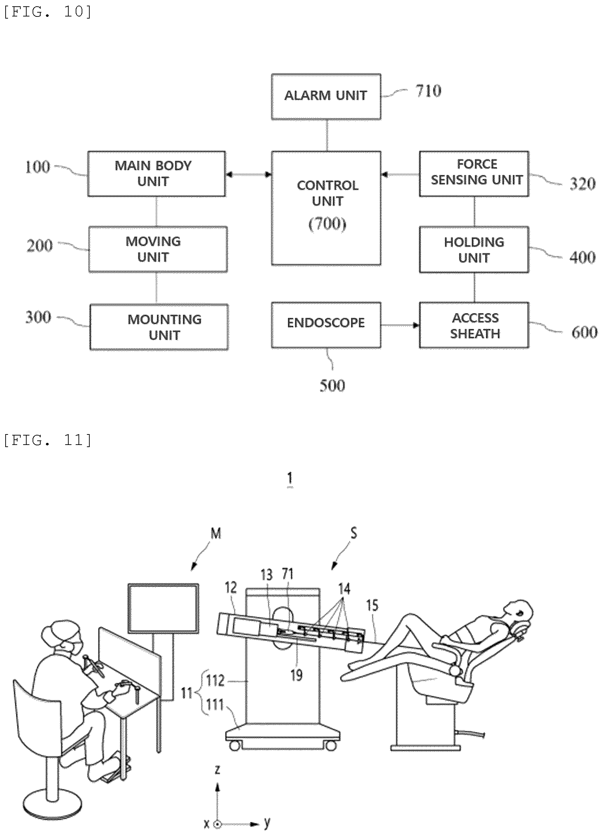

In addition, referring to FIG. 10, the surgical robot according to an embodiment of the present disclosure may include a control unit 700. The control unit 700 receives a measured value measured from the force sensing unit 320 and compares the measured value with a preset reference value. Furthermore, when the measured value is larger than the reference value, the control unit 700 may stop the operation of the surgical robot or may notify a user (a doctor and so on) by sounding an alarm through a separate alarm unit 710 as a subsequent action.

In addition, in the present disclosure, when a measured value of a force acting on the access sheath 600 is measured by the force sensing unit 320 and the measured value exceeds the reference value, the mounting position of the access sheath 600 is capable of being moved by changing setting positions of the force sensing unit 320 and the support holder 410 that supports the access sheath 600 in order to reduce the measured force.

In other words, in the control unit 700, the measured value of the force applied to the access sheath 600 provided by the force sensing unit 320 is compared with the reference value. Furthermore, as a result of comparison, when the measured value is larger than the reference value, the actuator is controlled so that the measured value does not exceed the reference value, and the assembly of the support holder 410 and the force sensing unit 320 may be moved within the moving unit 200.

For example, in the surgical robot according to an embodiment of the present disclosure, in order to remove the stone S in the patient's kidney K, the access sheath 600 may be first inserted into the inner portion of the ureter U, and then the endoscope 500 may be inserted through the inner portion of the access sheath 600 to access the stone S inside the kidney K.

The endoscope 500 includes the overtube 520, and the end portion of the overtube 520 is bent according to the operation of the endoscope 500, so that the endoscope 500 may be moved toward a place where the stone is positioned.

The endoscope 500 is in a state in which the endoscope is mounted on the mounting unit 300 of the surgical robot, and the overtube 520 of the endoscope 500 is mounted through the inlet member 610 and the protective tube 620 of the access sheath 600 held on the support holder 410 of the holding unit 400. At this time, the mounting unit 300 is capable of reciprocating in the moving unit 200 in the longitudinal direction, so that the overtube 520 of the endoscope 500 is capable of reciprocating inside the access sheath 600.

The ureter U is not formed only in a rectilinear shape. Therefore, when the access sheath 600 is inserted into the ureter U, a curved section may occur. Therefore, in a process of inserting the overtube 520 of the endoscope 500 into the access sheath 600, a large friction may occur due to the curved section of the access sheath 600. At this time, a force applied to the access sheath 600 when the insertion operation of the overtube 520 is performed may be measured by the force sensing unit 320.

In addition, in a state in which the endoscope 500 is not inserted into the access sheath 600, when the access sheath 600 is inserted into the ureter U, the ureter may press the access sheath.

In other words, after the access sheath 600 is entered into the inlet of the ureter before the endoscope 500 is inserted into the access sheath 600, when the holding of the access sheath 600 is performed while the access sheath 600 is in an appropriate state in which the access sheath 600 is not properly aligned to the support holder 410, the ureter is folded when the access sheath 600 pushes the ureter. At this time, a pushing force may be applied to the access sheath 600 during a process of inserting the access sheath 600 into the ureter. The inner wall of the ureter may be damaged by such a force.

Therefore, when the pushing force is applied to the access sheath 600 due to the ureter, the pushing force is transmitted to the force sensing unit 320 through the support holder 410 that is held, and the pushing force by the ureter is capable of being measured.

the measured force may be identified, and the position of the access sheath 600 may be set again so that the measured value of the pushing force becomes zero. Then, the endoscope 500 may be inserted into the inner portion of the set access sheath 600, and the surgical operation may be performed.

In addition, in a process of performing the surgical operation after the setting of the access sheath 600 is performed, the ureter may be moved by the patient's breath, and then a force pushing the access sheath 600 may be applied by the access sheath 600 in a fixed state and the ureter that is moved. Therefore, in such a situation, the force pushing the access sheath 600 by the ureter may be measured by the force sensing unit 320.

The laser part or the basket may be inserted into the overtube 520 of the endoscope 500. Furthermore, in a situation in which the laser part is inserted into the overtube 520 of the endoscope 500, when the overtube 520 is inserted into the inner portion of the access sheath 600, the laser part may protrude from the end portion of the overtube 520, and the laser part may break the stone while being spaced apart from the stone by a predetermined distance.

After the stone S of the kidney K is broken, as a subsequent action, the stone may be captured and may be extracted outside the access sheath 600 through the endoscope 500 provided with the overtube 520 having the basket 550.

However, the present disclosure is not limited thereto, and the surgical operation may be performed by being provided with an endoscope in which the laser part and the basket 550 are provided inside a channel of the overtube 520.

At this time, the guide tube 530 may be inserted into an internal space of the overtube 520, and is capable of being moved. The guide tube 530 may guide the wire 540 and the basket 550 to a place where the stone is positioned.

Since the guide tube 530 may be a part of the endoscope 500, an end portion of the guide tube 530 may be bent according to the operation of the endoscope 500, so that the end portion of the guide tube 530 may be moved toward the place where the stone is positioned.

The wire 540 may be inserted into an inner portion of the guide tube 530, and is capable of being moved.

In addition, the wire 540 may be moved backward relative to the guide tube 540 until the basket 550 is in a state in which the basket 500 is caught in the front of the guide tube 530 and is no longer capable of moving backward.

The basket 550 is provided in the front of the wire 540, is capable of holding the stone while being converted into an expanded state or a contracted state.

The basket 550 may be formed of a material that is restored to an original shape thereof when there is no external force applied to the material.

An imaging part 560 is provided on a front end portion of the guide tube 530, and may photograph the stone held by the basket 550.

The imaging part 560 may include a high-resolution ultra-small endoscopic camera.

The imaging part 560 may rotate the basket 550 in which the stone is held, and may photograph the entire shape of the outer circumference of the stone. Therefore, when the imaging part 560 is capable of identifying the entire shape of the stone that is held inside the basket 550 at once, the imaging part 560 may perform the photographing at once without rotating the basket 550.

Here, according to an embodiment of the present disclosure, in a state in which the basket 550 holds the stone, when the basket 550 is moved backward through the inner portion of the access sheath 600, the size of the stone may be a size that is difficult to pass through the internal space of the access sheath 600 or may be a size that is capable of passing through the internal space of the access sheath 600 but does not pass smoothly.

A direction in which the basket 550 is moved backward through the inner portion of the access sheath 600 may be a direction in which the basket 550 is moved outward from the kidney.

In this situation, a backward force of the overtube 520 of the endoscope 500 that is moved backward is transmitted to the access sheath 600, and the access sheath 600 may receive a force to be moved backward from the original position thereof.

At this time, the support holder 410 of the holding unit 400 holding the access sheath 600 receives the backward force and, accordingly, the arm part 412 of the support holder 410 is moved, so that the force is capable of being measured by the force sensing unit 320 that is in the fixed state.

In addition, in a process of removing the stone captured by the basket 550, attempting to forcibly withdraw (extract) the stone even though the stone is stuck in the access sheath 600 may result in breaking of the basket 500 or the wire 540. When such a situation occurs, it may be very difficult to resolve. Therefore, by measuring the pulling force acting as the stone is stuck in the access sheath 600 by using the force sensing unit 320, a subsequent action is capable of being performed when the measured value exceeds the reference value.

As such, two directions of forces may be applied to the access sheath 600 according to the movement of the overtube 520, and the force sensing unit 320 may measure the force in the two directions acting on the access sheath 600.

When a direction in which the overtube 520 is inserted into the inner portion of the access sheath 600 is referred to as a first direction and a direction in which the overtube 520 is withdrawn from the inner portion of the access sheath 600 is referred to as a second direction, a force in the first direction may be a force generated during the process of inserting the overtube 520 into the inner portion of the access sheath 600, and a force in the second direction may be a force generated during the process of withdrawing the overtube 520 to the outside of the access sheath 600.

In other words, the force in the first direction may be a pushing force generated in the process of inserting the overtube 520 into the inner portion of the access sheath 600, and the force in the second direction may be a pulling force generated in the process of withdrawing the overtube 520 to the outside of the access sheath 600.

In addition, when smooth insertion of the overtube 520 is not realized during the process of inserting the overtube 520 into the inner portion of the access sheath 600, an operation of moving the overtube 520 backward is also performed so that the overtube 520 is moved forward and backward, thereby being capable of inserting the overtube 520 into the inner portion of the access sheath 600.

Therefore, the force in the first direction may be a force generated by friction or a jam generated by the curved section when the overtube 520 is inserted into the inner portion of the access sheath 600, and the force in the second direction may be a force that is applied to the access sheath 600 by the stone when the stone that is broken in the kidney is moved backward from the inner portion of the access sheath 600 and is withdrawn outside the patient's body.

In addition, in a state in which the endoscope 500 is not inserted into the inner portion of the access sheath 600, a pushing force from the ureter may be applied to the access sheath 600. As such, the force applied to the access sheath 600 by the ureter is capable of being measured by the force sensing unit 320.

As such, when a force acting on the access sheath 600 or the endoscope 500 is measured by the force sensing unit 320, information on the measured value may be applied to the control unit 700. The control unit 700 compares the measured value measured by the force sensing unit 320 with the reference value. Furthermore, when the measured value is larger than the reference value, the control unit 700 guides the result through the alarm unit 710 and temporarily stops the operation of the surgical robot. Then, after the size of the stone is changed to a size capable of being smoothly passing through the access sheath 600 or the ureter by performing the stone breaking operation again, the operation of extracting the stone to the outside of the body from the inner portion of the kidney through the basket 550 may be performed again.

In the surgical robot according to an embodiment of the present disclosure, the moving unit 200 is rotated on the main body unit 100, the mounting unit 300 is reciprocated on the moving unit 200, and the endoscope 500 is capable of being rotated by the mounting part 310 provided on the mounting unit 300. Therefore, an appropriate operation is capable of being realized by accurately measuring a force generated during a surgical operation by the force sensing unit 320 and the surgical operation of removing the stone in the patient's kidney is clearly realized at the same time, so that the surgical operation is capable of being performed more safely.

In an embodiment of the present disclosure, a surgical operation of removing a kidney stone is described, but the present disclosure is not limited thereto, and the present disclosure is capable of being applied to various types of surgical operations using an endoscope, such as a surgical operation of removing a bile duct stone and so on.

Features, structures, effects, and so on described in the embodiments above are included in at least one embodiment, and are not necessarily limited to only one embodiment. Furthermore, the features, structures, effects, and so on illustrated in each embodiment may be combined or modified for other embodiments by a person having ordinary knowledge in the field to which the embodiments belong. Therefore, the contents related to these combinations and modifications should be construed as being included in the scope of the present disclosure.

In addition, while the present disclosure has been described with reference to exemplary embodiments thereof, it is clearly understood that the embodiments are only examples by way of illustration and are not intended to limit the present disclosure. Thus, it should be understood that those skilled in the art to which the present disclosure belongs can make a variety of modifications and applications of the present disclosure, which are not described here, without departing from the essential features thereof. For example, the respective components that are shown in detail in the embodiments may be modified to be executed. In addition, differences in relation to the modifications and applications should be construed to be included in the scope of the present disclosure as claimed in the appended claims.

----------------------------------------------------- ------------------------

Hereinafter, embodiments illustrated in FIG. 11 to FIG. 16 will be described.

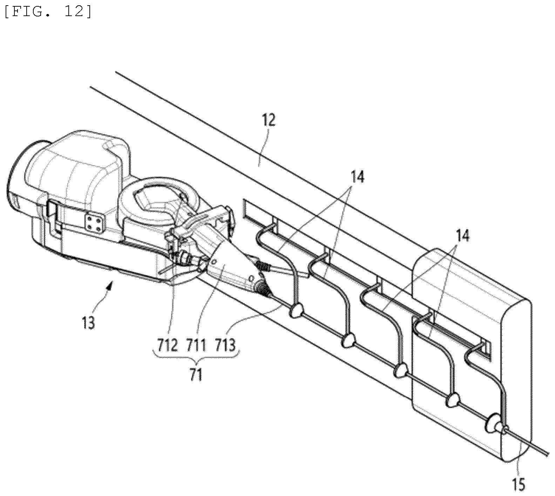

FIG. 11 is a view illustrating a surgical robot according to an embodiment, and FIG. 12 is a perspective view illustrating a driving part of a slave device according to an embodiment.

Referring to FIG. 11 and FIG. 12, an operator according to an embodiment may perform a surgical operation by using a surgical robot 1. The surgical robot 1 may include a master device M and a slave device S.

The master device M may be controlled by the operator. For example, the master device M may include a screen and a controller. The operator may control the slave device S connected to the master device M by using the controller, and may check a progress of the surgical operation in real time through the screen. The master device M and the slave device S may be connected to each other in a wired or wireless manner. The operator may directly input a command on the screen in order to control the slave device S. For example, the operator may input values such as a height, a rotation angle, and so on of the slave device S into the screen. Meanwhile, unlike the drawings of the present specification, the screen and the controller of the master device M may be provided as an integrated unit.

The slave device S may perform an operation required for the surgical operation by receiving a signal from the master device M. The slave device S may include a surgical tool configured to be inserted into the patient's body. For example, the slave device S may include an endoscopic device, a laser for breaking a kidney stone, a basket for holding a broken kidney stone, and so on. The slave device S may include a support part 11, a driving part 12, a surgical robot 13, an endoscope holder 14, an access sheath 15, and an endoscopic device 71.

The support part 11 may be provided such that the support part 11 is capable of being moved with respect to the ground. The support part 11 may include a support base 111 to which at least one wheel is attached, and may include a support body 112 connected to the support base 111. The support body 112 may be moved relative to the support base 111 by receiving a signal from the master device M. For example, the support body 112 may be rotated with respect to the support base 111 in a z-axis as a rotation axis, or may be moved translationally in an x-axis direction and a y-axis direction.

The driving part 12 is supported by the supporting part 11, and may be moved relative to the supporting part 11. For example, the driving part 12 may be rotated with respect to the support body 112 in the x-axis as a rotation axis by receiving a signal from the master device M, or may be moved translationally in a z-axis direction.

The surgical robot 13 may be provided such that the surgical robot 13 is connected to a rail 19 formed in the driving part 12 and the surgical robot 13 is capable of being slid in a longitudinal direction of the driving part 12. The surgical robot 13 may support the endoscopic device 71, and may control the endoscopic device 71 by receiving a signal from the master device M. For example, the surgical robot 13 may move a handle 712 of the endoscopic device 71, the handle 712 being configured to control a direction to which an endoscope 713 of the endoscopic device 71 is bent. In a state in which the endoscopic device 71 is supported on the surgical robot 13, a relative movement of the endoscopic device 71 with respect to the surgical robot 13 may be limited. Therefore, an error generated from a movement of the endoscopic device 71 may be reduced, and the stability and accuracy of the surgical operation may be increased.

The endoscope holder 14 may support the endoscope 713 of the endoscopic device 71. The endoscope holder 14 may prevent buckling of the endoscope 713 of the endoscopic device 71 when the surgical robot 13 is slid or rotated with respect to the driving part 12 of the surgical robot 13. For example, the endoscope holder 14 may be provided with a plurality of endoscope holders 14 along a longitudinal direction of the endoscope 713 of the endoscopic device 71, and may be spaced apart from each other. For example, the plurality of endoscope holders 14 is capable of being slid in one direction along the longitudinal direction of the driving part 12.

The access sheath 15 is connected to an end portion of the driving part 12, and may be inserted into the patient's body during the surgical operation. The endoscope 713 of the endoscopic device 71 may reach a surgical site of the patient by passing through the access sheath 15.

The endoscopic device 71 mounted on the surgical robot 13 may be an endoscopic device configured to control bending of the endoscope 713 from a relative movement of the handle 712 with respect to a main body 711 of the endoscopic device 71. For example, when the handle 712 of the endoscopic device 71 is moved in any one direction with respect to the main body 711 of the endoscopic device 71, the endoscope 713 of the endoscopic device 71 may be bent in a left direction. Furthermore, when the handle 712 is moved in a direction opposite to any one direction, the endoscope 713 of the endoscopic device 71 may be bent in a right direction.

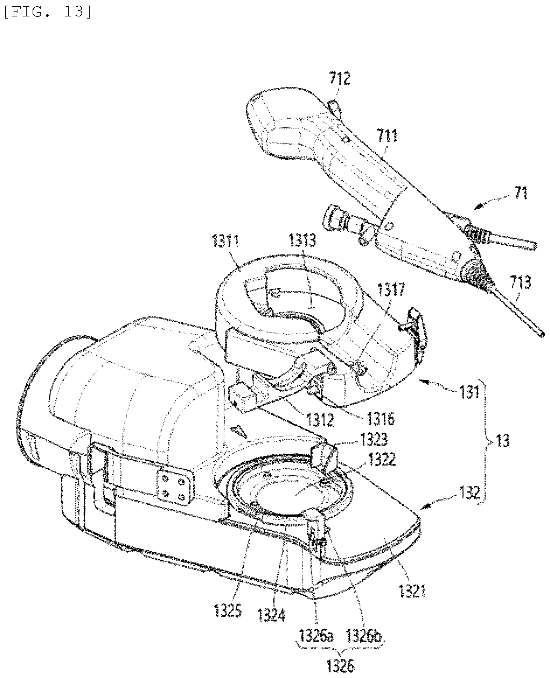

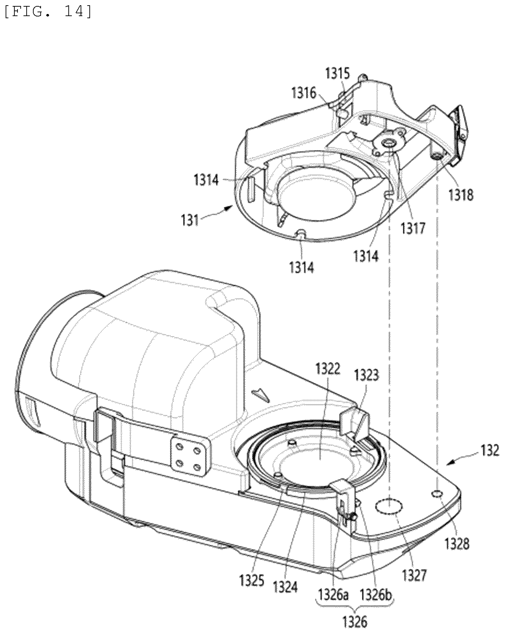

FIG. 13 is a perspective view illustrating an endoscopic device, a coupler, and a mount manufactured by Boston Scientific according to an embodiment, and FIG. 14 is a perspective view illustrating the coupler and the mount according to an embodiment.

Referring to FIG. 13 and FIG. 14, the surgical robot 13 according to an embodiment may include a coupler 131 and a mount 132. The coupler 131 may support the endoscopic device 71, and may fix a portion of a drape (not illustrated) to the mount 132. Here, the drape refers to a vinyl that separates the master device and the slave device in the surgical robot from microorganisms and other contamination factors. After a first end of the drape is disposed between the coupler 131 and the mount 132, the coupler 131 is mounted on the mount 132, so that the portion of the drape may be pressed by the coupler 131 and the mount 132. By this arrangement, the portion of the drape may be fixed to the surgical robot 13, and the surgical robot 13 may be completely covered by the remaining portion of the drape. The coupler 131 may be a disposable configuration replaced for hygiene after the surgical operation is finished.

The coupler 131 may have various shapes such that the coupler 131 accommodates the endoscopic device 71 manufactured by various manufacturers including Boston Scientific, Karl Storz, and OTU Medical. The specifications of the endoscopic device 71 manufactured by various manufacturers may vary. For example, a position of the handle 712 with respect to the main body 711 of the endoscopic device 71, a direction to which the endoscope 713 extends from the main body 711, and so on may vary. When the surgical robot 13 having a predetermined shape is used, it may be difficult to use the endoscopic device 71 having various specifications interchangeably. By replacing the coupler 131 having different shapes with the mount 132 having a predetermined shape, the operator is capable of using various types of the endoscopic device 71. Hereinafter, for convenience of description, the coupler 131 that accommodates the endoscopic device 71 manufactured by Boston Scientific will be described. The coupler 131 may include a coupler body 1311, a cover 1312, a main hole 1313, and a guide protrusion 1314.

The endoscopic device 71 may be disposed on the coupler body 1311. The coupler body 1311 may include a groove recessed along an edge of the endoscopic device 71 so that the endoscopic device 71 is accommodated in the groove. In a state in which the endoscopic device 71 is accommodated in the coupler body 1311, a rotation of the endoscopic device 71 with respect to the coupler body 1311 may be limited.

The cover 1312 may have a first end portion hingedly connected to the coupler body 1311, and may prevent the endoscopic device 71 from being detached from the coupler body 1311. After the endoscopic device 71 is accommodated in the coupler body 1311, a second end portion of the cover 1312 may be fixed to the coupler body 1311. In such a state, the endoscopic device 71 is pressed by the cover 1312, and a relative movement of the cover 1312 with respect to the coupler body 1311 may be limited.

A main hole 1313 may be formed through the coupler body 1311. The handle 712 of the endoscopic device 71 may be accommodated in the main hole 1313. The main hole 1313 may be formed along a circumference around a first axis. The main hole 1313 may be a space in which the handle 712 is capable of being moved with respect to the main body 711 of the endoscopic device 71. Since the main body 711 of the endoscopic device 71 is fixed by the coupler body 1311 and the cover 1312, bending of the endoscope 713 of the endoscopic device 71 is capable of being controlled by moving the handle 712 with respect to the main body 711.

The mount 132 may include a mount body 1321, a rotor 1322, a handle holder 1323, a vertical extension part 1324, and a plurality of guide grooves 1325.

The mount body 1321 may be connected to the rail formed on the driving part. On a first surface of the mount body 1321, the rotor 1322 capable of being rotated with respect to the first axis may be provided. The rotor 1322 may be rotated in a clockwise direction and a counter-clockwise direction by receiving a signal from the master device.

The handle holder 1323 is connected to the rotor 1322, and may be connected to the handle 712 of the endoscopic device 71. For example, the handle holder 1323 may be hingedly coupled to an edge of the rotor 1322. The handle 712 of the endoscopic device 71 may be positioned between a pair of pressing surfaces of the handle holder 1323. The pair of pressing surfaces facing each other may respectively press a first side and a second side of the handle 712 of the endoscopic device 71. For example, when the rotor 1322 is rotated in the clockwise direction, any one of the pair of pressing surfaces presses the first side of the handle 712 of the endoscopic device 71, and the handle 712 of the endoscopic device 71 may be rotated in the clockwise direction. On the other hand, when the rotor 1322 is rotated in the counter-clockwise direction, the other one of the pair of pressing surfaces presses the second side of the handle 712 of the endoscopic device 71, and the handle 712 of the endoscopic device 71 may be rotated in the counter-clockwise direction.

In an embodiment, the handle holder 1323 may be provided as a replaceable part. For example, the handle 712 of the endoscopic device 71 manufactured by various manufacturers may have different widths from each other. Due to a distance existing between the handle 712 of the endoscopic device 71 and the pair of pressing surfaces of the handle holder 1323, a delay time may occur in pressing the handle 712 of the endoscopic device 71 by the handle holder 1323 when the handle holder 1323 in a stationary state is rotated in any one direction. By using the respective handle holders 1323 fixed to each of the handles 712 having different widths, an error generated from the delay time may be reduced, and the maintenance cost and the repair cost of the surgical robot 13 may be reduced.

The vertical extension part 1324 protrudes from the mount body 1321, and may be formed along a circumference of the rotor 1322 around a rotation axis of the rotor 1322. For example, the vertical extension part 1324 may be formed along an edge of the rotor 1322, and may prevent foreign substances from penetrating to a distance between the mount body 1321 and the rotor 1322. The plurality of guide grooves 1325 may be formed on an outer circumferential surface of the vertical extension part 1324.

The plurality of guide grooves 1325 may be formed by being spaced apart from each other around the rotation axis of the rotor 1322. Each of the guide grooves 1325 may be formed along the circumference of the rotor 1322 around the rotation axis of the rotor 1322. From such a structure, after the guide protrusion 1314 of the coupler 131 is inserted into the guide groove 1325, the coupler 131 is rotated along a direction in which the guide groove 1325 is formed, and the coupler 131 may be mounted on the mount 132. In a state in which the coupler 131 is mounted on the mount 132, an upward and downward movement of the coupler 131 with respect to the mount 132 may be limited.

In the drawings of the present specification, it is illustrated that three guide protrusions 1314 and three guide grooves 1325 are provided, but the number of guide protrusions 1314 and the number of guide grooves 1325 are not limited thereto.

In the state in which the coupler 131 is mounted on the mount 132, the rotation of the coupler 131 with respect to the mount 132 may be limited by a hook 1326 and an elastic protrusion 1316.

The hook 1326 may include a hook body 1326a supported by the mount body 1321, and may include a hook head 1326b that protrudes from the hook body 1326a. In the state in which the coupler 131 is mounted on the mount 132, the hook head 1326b may be accommodated in a hook accommodating groove 1315 formed in the coupler body 1311 facing the hook body 1326a, and the hook head 1326b may be caught on the coupler body 1311. In a situation in which the coupler body 1311 is intended to be rotated in a direction in which the hook accommodating groove 1315 is moved away from the hook head 1326b, the coupler body 1311 is attached to the hook head 1326b, so that the rotation of the coupler body 1311 may be limited.

The elastic protrusion 1316 protrudes from the coupler body 1311, and may be provided such that the elastic protrusion 1316 is compressed when the elastic protrusion 1316 is pressed, and that the elastic protrusion 1316 is capable of being returned to an original position thereof by an elastic force when the elastic protrusion 1316 is not pressed. In a state in which the hook head 1326b is caught on the coupler body 1311, the elastic protrusion 1316 may be in a state in which the elastic protrusion 1316 is pressing the hook body 1326a. In a situation in which the coupler body 1311 is intended to be rotated in a direction in which the hook accommodating groove 1315 is moved closer to the hook head 1326b, the rotation of the coupler body 1311 may be limited due to a restoring force of the elastic protrusion 1316. The state in which the hook head 1326b is caught on the coupler body 1311 may be maintained. Such a state may be referred to as a "completely fastened state".

The coupler 131 may further include at least one of a first detection target 1317 and a second detection target 1318. The operator may determine, through the first detection target 1317, whether the endoscopic device 71 is supported on the coupler 131. The operator may check, through the second detection target 1318, whether the coupler 131 is fixed to the mount 132.

The first detection target 1317 may be formed through the coupler body 1311 in a direction in which the endoscopic device 71 is accommodated in the coupler 131. An upper portion of the first detection target 1317 may be present on a surface of the coupler body 1311 in which the endoscopic device 71 is disposed, and a lower portion of the first detection target 1317 may be present on a surface opposite to the surface of the coupler body 1311 in which the endoscopic device 71 is disposed. For example, the upper portion of the first detection target 1317 may include an elastic material, and the lower portion of the first detection target 1317 may include metal.

In a state in which the endoscopic device 71 is not accommodated in the coupler 131, the upper portion of the first detection target 1317 may be in a state in which the upper portion of the first detection target 1317 protrudes from the coupler body 1311. In a situation in which the endoscopic device 71 is accommodated in the coupler 131 while the coupler 131 is in a state in which the coupler 131 is mounted on the mount 132, the upper portion of the first detection target 1317 may be pressed by the endoscopic device 71, and the lower portion of the first detection target 1317 may be moved toward the mount 132. A first sensor 1327 formed in the mount body 1321 may detect that the metal contained in the lower portion of the first detection target 1317 is disposed within a predetermined distance, and may generate a signal. For example, the first sensor 1327 may be disposed inside the mount body 1321. Meanwhile, in a situation in which the first detection target 1317 is no longer pressed by the endoscopic device 71, the first detection target 1317 may be returned to an original position thereof by a restoring force.

The second detection target 1318 may be disposed on a surface of the coupler body 1311 facing the mount 132. For example, the second detection target 1318 may be metal. In the completely fastened state in which the coupler 131 is guided by the guide grooves 1325 of the mount 132, the coupler 131 is connected to the vertical extension part 1324, and the hook head 1326b of the mount 132 is caught on the coupler body 1311, the second detection target 1318 may overlap a second sensor 1328 formed in the mount body 1321 on the basis of a direction in which the endoscopic device 71 is accommodated in the coupler 131. The second sensor 1328 may detect that the second detection target 1318 is disposed within a predetermined distance, and may generate a signal. For example, the second sensor 1328 may be disposed inside the mount body 1321.

Meanwhile, in a situation in which the first detection target 1317 uses metal and the second detection target 1318 uses metal as well, the operator may appropriately set a sensing range of the first sensor 1327 and a sensing range of the second sensor 1328 so that the first sensor 1327 does not recognize the second detection target 1318 or the second sensor 1328 does not detect the first detection target 1317.

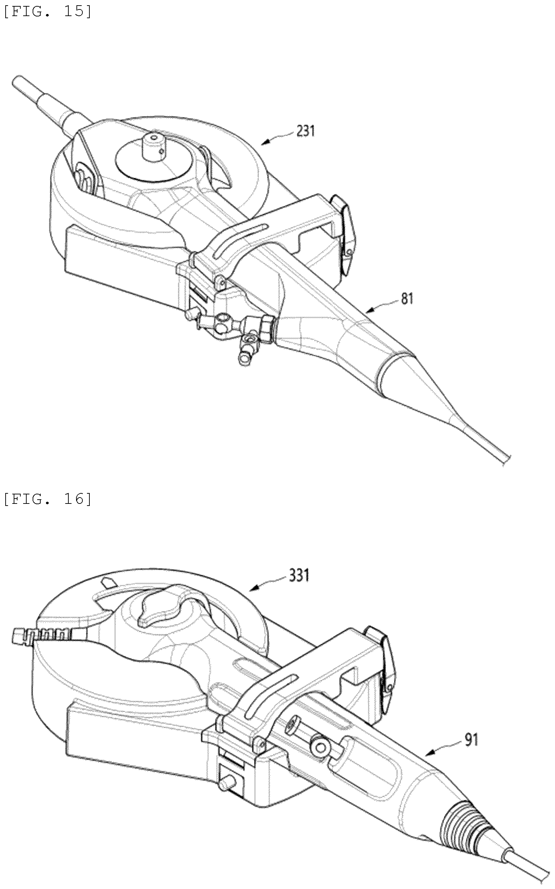

FIG. 15 is a perspective view illustrating a coupler supporting an endoscopic device manufactured by Karl Storz according to an embodiment, and FIG. 16 is a perspective view illustrating a coupler supporting an endoscopic device manufactured by OTU Medical according to an embodiment.

Referring to FIG. 15 and FIG. 16, a coupler 231 for supporting an endoscopic device 81 manufactured by Karl Storz according to an embodiment, and a coupler 331 for supporting the endoscopic device 71 manufactured by OTU Medical may be designed to have different shapes.

A coupler (for example: the coupler 131 in FIG. 13, the coupler 231 in FIG. 15, and the coupler 331 in FIG. 16) may be designed in consideration of not only a shape of an endoscopic device (for example: the endoscopic device 71 in FIG. 13, the endoscopic device 81 in FIG. 15, and the endoscopic device 91 in FIG. 16) but also a width of the handle of each endoscopic device 71, 81, and 91, a driving position of the handle of each endoscopic device 71, 81, and 91. For example, a position, a depth, and so on of a groove formed in the coupler body may be designed differently according to a shape of the main body of each endoscopic device 71, 81, and 91. For example, a position, a width, and so on of the main hole formed in the coupler body may be designed differently according to a moving range of the handle and a length to which the handle protrudes from the main body.

In a state in which the endoscope is not bent, an initial position which is the position of the handle with respect to the main body may vary. In such a situation, after the rotor of the mount is rotated in advance so that the handle holder is moved to the initial position of the handle, the handle holder may be fixed to the handle. Each length of the main body of each endoscopic device 71, 81, and 91 manufactured by various manufacturers may vary. In a state in which each endoscopic device 71, 81, and 91 is supported by the surgical robot, a distance between the main body of the endoscope and the endoscope holder (hereinafter, referred to as a "first endoscope holder") that is positioned closest to the main body among the plurality of endoscope holders may vary for each endoscopic device 71, 81, and 91.

The buckling of the endoscope may increase as an angle between a direction in which the endoscope of each endoscopic device 71, 81, and 91 enters the first endoscope holder and a direction in which the plurality of endoscope holders is arranged in a row increases. When the endoscope that extends from the main body of each endoscopic device 71, 81, and 91 passes through the plurality of endoscope holders rectilinearly without bending, the buckling of the endoscope may be reduced. When the buckling of the endoscope occurs, it is difficult for the endoscope to reach the patient's affected area, and an error may occur between the user's operation and the movement of the endoscope.

In order to reduce the buckling of the endoscope of each endoscopic device 71, 81, and 91, each angle at which each endoscopic device 71, 81, and 91 is accommodated in the surgical robot may vary. In other words, in a state in which each endoscopic device 71, 81, and 91 is accommodated in each coupler 131, 231, and 331 and each coupler 131, 231, and 331 is in the completely fastened state with respect to the mount, an angle between a longitudinal direction of each endoscopic device 71, 81, and 91 and a longitudinal direction of the driving part may vary. For example, by differently designing the position of the guide protrusion formed on the coupler body, the angle between the longitudinal direction of each endoscopic device 71, 81, and 91 and the longitudinal direction of the driving part may be adjusted.

Meanwhile, a modeling program may be used to determine the arrangement of each endoscopic device 71, 81, and 91 minimizing the buckling of the endoscope. For example, by modeling the shape of each endoscopic device 71, 81, and 91, values such as the length of the main body, the angle of the endoscope that protrudes from the main body, the distance between the main body and the first endoscope holder, and so on may be calculated, and an optimal arrangement position of each endoscopic device 71, 81, and 91 capable of minimizing the buckling may be determined.

----------------------------------------------------- **------------------------**

### <ADDITIONAL EMBODIMENTS #1-1>

Embodiment 1. A surgical robot assembly comprising:
an access sheath configured to be inserted into a subject;
an endoscope comprising an overtube configured to be inserted into the access sheath;
a surgical tool configured to be inserted into the overtube; and
a sensing unit configured to detect a presence, absence, or amount of a force applied to the access sheath.

Embodiment 2. The surgical robot assembly according to the preceding embodiment, wherein the overtube is coupled to an end of the endoscope.

Embodiment 3. The surgical robot assembly according to any one of the preceding embodiments, wherein the subject is human.

Embodiment 4. The surgical robot assembly according to any one of the preceding embodiments, wherein the subject is an animal.

Embodiment 5. The surgical robot assembly according to any one of the preceding embodiments, wherein the presence, absence, or amount of the force is detected.

Embodiment 6. The surgical robot assembly according to any one of the preceding embodiments, wherein the endoscope is configured to enter and/or exit the subject.

Embodiment 7. The surgical robot assembly according to any one of the preceding embodiments, wherein a stone is removed from the subject by the surgical robot assembly.

Embodiment 8. The surgical robot assembly according to any one of the preceding embodiments, wherein the access sheath comprises a first end and a second end opposite to the first end, and the first end is provided with an inlet.

Embodiment 9. The surgical robot assembly according to any one of the preceding embodiments, wherein the inlet is formed in a conical shape.

Embodiment 10. The surgical robot assembly according to any one of the preceding embodiments, further comprising a support holder, wherein the support holder is operatively coupled to the inlet.

Embodiment 11. The surgical robot assembly according to any one of the preceding embodiments, wherein the support holder comprises a holding member and an arm member, wherein the holding member is configured to be coupled to the inlet of the access sheath, and wherein a first end of the arm member is coupled to the holding member and a second end of the arm member is coupled to the sensing unit.

Embodiment 12. The surgical robot assembly according to any one of the preceding embodiments, wherein the arm member comprises a curved portion, and wherein the arm member is configured to act as a hinge point.

Embodiment 13. The surgical robot assembly according to any one of the preceding embodiments, wherein the overtube is capable of being moved within the access sheath in a first direction and a second direction that is opposite to the first direction, wherein, when the overtube is moved within the access sheath in the first direction, a first force is generated, and wherein, when the overtube is moved within the access sheath in the second direction, a second force is generated.

Embodiment 14. The surgical robot assembly according to any one of the preceding embodiments, wherein the sensing unit is configured to measure each of the first force and the second force.

Embodiment 15. The surgical robot assembly according to any one of the preceding embodiments, wherein, when the overtube is moved in the first direction or the second direction, the first force or the second force is transmitted to the arm member.

Embodiment 16. The surgical robot assembly according to any one of the preceding embodiments, further comprising a guide tube, a wire, and a basket disposed at an end of the wire, wherein the wire is configured to be inserted through the guide tube, and wherein the guide tube is provided as a tube configured to be inserted through the access sheath.

Embodiment 17. The surgical robot assembly according to any one of the preceding embodiments, further comprising a control unit configured to analyze the force detected by the sensing unit, wherein, when the force exceeds a threshold value, the control unit operates an alarm unit.

Embodiment 18. The surgical robot assembly according to any one of the preceding embodiments, wherein the threshold value is a predetermined force value.

Embodiment 19. The surgical robot assembly according to any one of the preceding embodiments, wherein the control unit is configured to compare the measured force detected by the sensing unit with the predetermined force value.

Embodiment 20. The surgical robot assembly according to any one of the preceding embodiments, wherein the endoscope is configured to be coupled to a moving unit, and wherein, during use of the endoscope, the endoscope is capable of being moved along the moving unit.

Embodiment 21. Use of the surgical robot assembly according to any one of the preceding embodiments for performing a surgical operation.

Embodiment 22. A surgical robot system comprising:
a master device provided with a screen and a controller;
a driving body;
a moving unit coupled to the driving body; and
a surgical robot assembly comprising an endoscope and configured to receive a signal from the master device, wherein the surgical robot assembly is configured to be moved with respect to a human body via the moving unit in response to the signal from the master device.

Embodiment 23. The surgical robot system according to embodiment 22, wherein the surgical robot assembly further comprises: an access sheath configured to be inserted into a subject;
an overtube configured to be inserted into the access sheath;
a surgical tool configured to be inserted into the overtube; and
a sensing unit configured to detect a presence, absence, or amount of a force applied to the access sheath.

Embodiment 24. The surgical robot system according to embodiment 22 or embodiment 23, wherein the overtube is coupled to an end of the endoscope.

Embodiment 25. The surgical robot system according to any one of embodiment 22 to embodiment 24, wherein the subject is human.

Embodiment 26. The surgical robot system according to any one of embodiment 22 to embodiment 24, wherein the subject is an animal.

Embodiment 27. The surgical robot system according to any one of embodiment 22 to embodiment 26, wherein the presence, absence, or amount of the force is detected.

Embodiment 28. The surgical robot system according to any one of embodiment 22 to embodiment 27, wherein the endoscope is configured to enter and/or exit the subject.

Embodiment 29. The surgical robot system according to any one of embodiment 22 to embodiment 28, wherein a stone is removed from the subject by the surgical robot assembly.

Embodiment 30. The surgical robot system according to any one of embodiment 22 to embodiment 29, wherein the access sheath comprises a first end and a second end opposite to the first end, and wherein the first end is provided with an inlet.

Embodiment 31. The surgical robot system according to any one of embodiment 22 to embodiment 30, wherein the inlet is formed in a conical shape.

Embodiment 32. The surgical robot system according to any one of embodiment 22 to embodiment 31, further comprising a support holder, wherein the support holder is operatively coupled to the inlet.

Embodiment 33. The surgical robot system according to any one of embodiment 22 to embodiment 32, wherein the support holder comprises a holding member and an arm member, wherein the holding member is configured to be coupled to the inlet of the access sheath, and wherein a first end of the arm member is coupled to the holding member and a second end of the arm member is coupled to the sensing unit.

Embodiment 34. The surgical robot system according to any one of embodiment 22 to embodiment 33, wherein the arm member comprises a curved portion, and wherein the arm member is configured to act as a hinge point.

Embodiment 35. The surgical robot system according to any one of embodiment 22 to embodiment 34, wherein the overtube is capable of being moved within the access sheath in a first direction and a second direction that is opposite to the first direction, wherein, when the overtube is moved within the access sheath in the first direction, a first force is generated, and wherein, when the overtube is moved within the access sheath in the second direction, a second force is generated.

Embodiment 36. The surgical robot system according to any one of embodiment 22 to embodiment 35, wherein the sensing unit is configured to measure each of the first force and the second force.

Embodiment 37. The surgical robot system according to any one of embodiment 22 to embodiment 36, wherein, when the overtube is moved in the first direction or the second direction, the first force or the second force is transmitted to the arm member.

Embodiment 38. The surgical robot system according to any one of embodiment 22 to embodiment 37, further comprising a guide tube, a wire, and a basket disposed at an end of the wire, wherein the wire is configured to be inserted through the guide tube, and wherein the guide tube is provided as a tube configured to be inserted through the access sheath.

Embodiment 39. The surgical robot system according to any one of embodiment 22 to embodiment 38, further comprising a control unit configured to analyze the force detected by the sensing unit, wherein, when the force exceeds a threshold value, the control unit operates an alarm unit.

Embodiment 40. The surgical robot system according to any one of embodiment 22 to embodiment 39, wherein the threshold value is a predetermined force value.

Embodiment 41. The surgical robot system according to any one of embodiment 22 to embodiment 40, wherein the control unit is configured to compare the measured force detected by the sensing unit with the predetermined force value.

Embodiment 42. The surgical robot system according to any one of embodiment 22 to embodiment 41, wherein the endoscope is configured to be coupled to a moving unit, and wherein, during use of the endoscope, the endoscope is capable of being moved along the moving unit.

Embodiment 43. Use of the surgical robot system according to any one of embodiment 22 to embodiment 42 for performing a surgical operation.

Embodiment 44. A method of operating a surgical robot, the method comprising: operating an endoscope assembly comprising an overtube, a surgical tool, an access sheath, and a sensing unit; and adjusting the endoscope assembly in response to a signal received from the sensing unit.

Embodiment 45. The method according to embodiment 44, wherein the access sheath comprises a first end and a second end opposite to the first end, and wherein the first end is provided with an inlet.

Embodiment 46. The method according to embodiment 44 or embodiment 45, wherein the endoscope assembly further comprises a support holder operatively coupled to the inlet, wherein the support holder is provided with a holding member and an arm member, wherein the holding member is configured to be coupled to the inlet of the access sheath, and wherein a first end of the arm member is coupled to the holding member and a second end of the arm member is coupled to the sensing unit.

Embodiment 47. The method according to any one of embodiment 44 to embodiment 46, wherein the operating of the endoscope assembly comprises: inserting the access sheath into a patient; and inserting the overtube through the access sheath, wherein, when the overtube is inserted through the access sheath in a first direction, a first force is generated, and wherein the sensing unit is configured to detect and measure the first force.

Embodiment 48. The method according to any one of embodiment 44 to embodiment 47, wherein, when the overtube is moved within the access sheath in a second direction, a second force is generated, and wherein the sensing unit is configured to detect and measure the second force.

Embodiment 49. The method according to any one of embodiment 44 to embodiment 48, wherein, when the endoscope assembly is operated, the first force or the second force is transmitted to the arm member.

Embodiment 50. The method according to any one of embodiment 44 to embodiment 49, wherein the endoscope assembly further comprises a guide tube, a wire, and a basket disposed at an end of the wire.

Embodiment 51. The method according to any one of embodiment 44 to embodiment 50, wherein the operating of the endoscope assembly comprises: inserting the guide tube through the access sheath; and inserting the wire and the basket through the guide tube.

Embodiment 52. The method according to any one of embodiment 44 to embodiment 51, wherein the endoscope assembly further comprises a control unit configured to analyze a force detected by the sensing unit.

Embodiment 53. The method according to any one of embodiment 44 to embodiment 52, wherein the adjusting of the endoscope assembly comprises: analyzing the force detected by the sensing unit; transmitting a signal to an alarm unit when the force exceeds a threshold force; and responding to an alarm generated by the alarm unit.

Embodiment 54. The method according to any one of embodiment 44 to embodiment 53, wherein the responding to the alarm generated by the alarm unit comprises stopping a movement of the overtube within the access sheath.

Embodiment 55. The method according to any one of embodiment 44 to embodiment 54, wherein the responding to the alarm generated by the alarm unit comprises changing a movement direction of the overtube within the access sheath.

Embodiment 56. The method according to any one of embodiment 44 to embodiment 55, wherein the threshold force is a predetermined force value, and wherein the control unit is configured to compare the measured force detected by the sensing unit with the predetermined force value.

Embodiment 57. The method according to any one of embodiment 44 to embodiment 56, further comprising providing a moving unit, wherein the endoscope assembly is operatively coupled to the moving unit such that the endoscope assembly is capable of being linearly moved along the moving unit.

Embodiment 58. The method according to any one of embodiment 44 to embodiment 57, wherein the moving unit is configured to be tilted with respect to a human body such that an angle of the endoscope assembly with respect to the patient is capable of being adjusted, and wherein the moving unit is capable of being moved vertically with respect to the human body, thereby being capable of adjusting a height of the endoscope assembly with respect to the patient.

Embodiment 59. The method according to any one of embodiment 44 to embodiment 58, wherein the operating of the endoscope assembly comprises: adjusting the height of the endoscope assembly by relatively adjusting a position of the moving human body vertically; and adjusting the angle of the endoscope assembly by adjusting an angle of the moving unit with respect to the human body.

### <FURTHER EMBODIMENTS #1-2>

Embodiment 1. A surgical robot assembly comprising:
An endoscope comprising an overtube configured to be inserted into a subject's ureter;
a surgical tool configured to be inserted into the overtube; and
a sensing unit configured to detect a presence, absence, or amount of a force applied to the ureter.

Embodiment 2. The surgical robot assembly according to the preceding embodiment, wherein the overtube is coupled to an end of the endoscope.

Embodiment 3. The surgical robot assembly according to any one of the preceding embodiments, wherein the subject is human.

Embodiment 4. The surgical robot assembly according to any one of the preceding embodiments, wherein the subject is an animal.

Embodiment 5. The surgical robot assembly according to any one of the preceding embodiments, wherein the presence, absence, or amount of the force is detected.

Embodiment 6. The surgical robot assembly according to any one of the preceding embodiments, wherein the endoscope is configured to enter and/or exit the subject.

Embodiment 7. The surgical robot assembly according to any one of the preceding embodiments, wherein a stone is removed from the subject by the surgical robot assembly.

Embodiment 8. The surgical robot assembly according to any one of the preceding embodiments, further comprising an access sheath configured to be inserted into the subject, wherein the overtube is configured to be inserted into the access sheath, wherein the access sheath comprises a first end and a second end that is positioned opposite to the first end, and wherein the first end is provided with an inlet.

Embodiment 9. The surgical robot assembly according to any one of the preceding embodiments, wherein the inlet is formed in a conical shape.

Embodiment 10. The surgical robot assembly according to any one of the preceding embodiments, further comprising a support holder, wherein the support holder is operatively coupled to the inlet.

Embodiment 11. The surgical robot assembly according to any one of the preceding embodiments, wherein the support holder comprises a holding member and an arm member, wherein the holding member is configured to be coupled to an inlet of the ureter or the inlet of the access sheath, and wherein a first end of the arm member is coupled to the holding member and a second end of the arm member is coupled to the sensing unit.

Embodiment 12. The surgical robot assembly according to any one of the preceding embodiments, wherein the arm member comprises a curved portion, and wherein the arm member is configured to act as a hinge point.

Embodiment 13. The surgical robot assembly according to any one of the preceding embodiments, wherein the overtube is capable of being moved within the ureter or the access sheath in a first direction and a second direction that is opposite to the first direction, wherein, when the overtube is moved within the ureter or the access sheath in the first direction, a first force is generated, and wherein, when the overtube is moved within the ureter or the access sheath in the second direction, a second force is generated.

Embodiment 14. The surgical robot assembly according to any one of the preceding embodiments, wherein the sensing unit is configured to measure each of the first force and the second force.

Embodiment 15. The surgical robot assembly according to any one of the preceding embodiments, wherein, when the overtube is moved in the first direction or the second direction, the first force or the second force is transmitted to the arm member.

Embodiment 16. The surgical robot assembly according to any one of the preceding embodiments, further comprising a guide tube, a wire, and a basket disposed at an end of the wire, wherein the wire is configured to be inserted through the guide tube, and wherein the guide tube is provided as a tube configured to be inserted through the ureter or the access sheath.

Embodiment 17. The surgical robot assembly according to any one of the preceding embodiments, further comprising a control unit configured to analyze the force detected by the sensing unit, wherein, when the force exceeds a threshold value, the control unit operates an alarm unit.

Embodiment 18. The surgical robot assembly according to any one of the preceding embodiments, wherein the threshold value is a predetermined force value.

Embodiment 19. The surgical robot assembly according to any one of the preceding embodiments, wherein the control unit is configured to compare the measured force detected by the sensing unit with the predetermined force value.

Embodiment 20. The surgical robot assembly according to any one of the preceding embodiments, wherein the endoscope is configured to be coupled to a moving unit, and wherein, during use of the endoscope, the endoscope is capable of being moved along the moving unit.

Embodiment 21. Use of the surgical robot assembly according to any one of the preceding embodiments for performing a surgical operation.

Embodiment 22. A surgical robot system comprising:
a master device provided with a screen and a controller;
a driving body;
a moving unit coupled to the driving body; and
a surgical robot assembly comprising an endoscope and configured to receive a signal from the master device, wherein the surgical robot assembly is configured to be moved with respect to a human body via the moving unit in response to the signal from the master device.

Embodiment 23. The surgical robot system according to embodiment 22, wherein the surgical robot assembly further comprises:
an overtube configured to be inserted into a subject's ureter;
a surgical tool configured to be inserted into the overtube; and
a sensing unit configured to detect a presence, absence, or amount of a force applied to the ureter.

Embodiment 24. The surgical robot system according to embodiment 22 or embodiment 23, wherein the overtube is coupled to an end of the endoscope.

Embodiment 25. The surgical robot system according to any one of embodiment 22 to embodiment 24, wherein the subject is human.

Embodiment 26. The surgical robot system according to any one of embodiment 22 to embodiment 24, wherein the subject is an animal.

Embodiment 27. The surgical robot system according to any one of embodiment 22 to embodiment 26, wherein the presence, absence, or amount of the force is detected.

Embodiment 28. The surgical robot system according to any one of embodiment 22 to embodiment 27, wherein the endoscope is configured to enter and/or exit the subject.

Embodiment 29. The surgical robot system according to any one of embodiment 22 to embodiment 28, wherein a stone is removed from the subject by the surgical robot assembly.

Embodiment 30. The surgical robot system according to any one of embodiment 22 to embodiment 29, further comprising an access sheath configured to be inserted into the subject, wherein the overtube is configured to be inserted into the access sheath, wherein the access sheath comprises a first end and a second end that is positioned opposite to the first end, and wherein the first end is provided with an inlet.

Embodiment 31. The surgical robot system according to any one of embodiment 22 to embodiment 30, wherein the inlet is formed in a conical shape.

Embodiment 32. The surgical robot system according to any one of embodiment 22 to embodiment 31, further comprising a support holder, wherein the support holder is operatively coupled to the inlet.

Embodiment 33. The surgical robot system according to any one of embodiment 22 to embodiment 32, wherein the support holder comprises a holding member and an arm member, wherein the holding member is configured to be coupled to an inlet of the ureter or the inlet of the access sheath, and wherein a first end of the arm member is coupled to the holding member and a second end of the arm member is coupled to the sensing unit.

Embodiment 34. The surgical robot system according to any one of embodiment 22 to embodiment 33, wherein the arm member comprises a curved portion, and wherein the arm member is configured to act as a hinge point.

Embodiment 35. The surgical robot system according to any one of embodiment 22 to embodiment 34, wherein the overtube is capable of being moved within the ureter or the access sheath in a first direction and a second direction that is opposite to the first direction, wherein, when the overtube is moved within the ureter or the access sheath in the first direction, a first force is generated, and wherein, when the overtube is moved within the ureter or the access sheath in the second direction, a second force is generated.

Embodiment 36. The surgical robot system according to any one of embodiment 22 to embodiment 35, wherein the sensing unit is configured to measure each of the first force and the second force.

Embodiment 37. The surgical robot system according to any one of embodiment 22 to embodiment 36, wherein, when the overtube is moved in the first direction or the second direction, the first force or the second force is transmitted to the arm member.

Embodiment 38. The surgical robot system according to any one of embodiment 22 to embodiment 37, further comprising a guide tube, a wire, and a basket disposed at an end of the wire, wherein the wire is configured to be inserted through the guide tube, and wherein the guide tube is provided as a tube configured to be inserted through the ureter or the access sheath.

Embodiment 39. The surgical robot system according to any one of embodiment 22 to embodiment 38, further comprising a control unit configured to analyze the force detected by the sensing unit, wherein, when the force exceeds a threshold value, the control unit operates an alarm unit.

Embodiment 40. The surgical robot system according to any one of embodiment 22 to embodiment 39, wherein the threshold value is a predetermined force value.

Embodiment 41. The surgical robot system according to any one of embodiment 22 to embodiment 40, wherein the control unit is configured to compare the measured force detected by the sensing unit with the predetermined force value.

Embodiment 42. The surgical robot system according to any one of embodiment 22 to embodiment 41, wherein the endoscope is configured to be coupled to a moving unit, and wherein, during use of the endoscope, the endoscope is capable of being moved along the moving unit.

Embodiment 43. Use of the surgical robot system according to any one of embodiment 22 to embodiment 42 for performing a surgical operation.

Embodiment 44. A method of operating a surgical robot, the method comprising: operating an endoscope assembly comprising an overtube, a surgical tool, and a sensing unit; and adjusting the endoscope assembly in response to a signal received from the sensing unit.

Embodiment 45. The method according to embodiment 44, wherein the endoscope assembly further comprises an access sheath, wherein the access sheath comprises a first end and a second end opposite to the first end, and wherein the first end is provided with an inlet.

Embodiment 46. The method according to embodiment 44 or embodiment 45, wherein the endoscope assembly further comprises a support holder operatively coupled to the inlet, wherein the support holder is provided with a holding member and an arm member, wherein the holding member is configured to be coupled to an inlet of a ureter or the inlet of the access sheath, and wherein a first end of the arm member is coupled to the holding member and a second end of the arm member is coupled to the sensing unit.

Embodiment 47. The method according to any one of embodiment 44 to embodiment 46, wherein the operating of the endoscope assembly comprises: optionally inserting the access sheath into a patient; and inserting the overtube through the ureter or the access sheath, wherein, when the overtube is inserted through the ureter or the access sheath in a first direction, a first force is generated, and wherein the sensing unit is configured to detect and measure the first force.

Embodiment 48. The method according to any one of embodiment 44 to embodiment 47, wherein, when the overtube is moved within the ureter or the access sheath in a second direction, a second force is generated, and wherein the sensing unit is configured to detect and measure the second force.

Embodiment 49. The method according to any one of embodiment 44 to embodiment 48, wherein, when the endoscope assembly is operated, the first force or the second force is transmitted to the arm member.

Embodiment 50. The method according to any one of embodiment 44 to embodiment 49, wherein the endoscope assembly further comprises a guide tube, a wire, and a basket disposed at an end of the wire.

Embodiment 51. The method according to any one of embodiment 44 to embodiment 50, wherein the operating of the endoscope assembly comprises: inserting the guide tube through the ureter or the access sheath; and inserting the wire and the basket through the guide tube.

Embodiment 52. The method according to any one of embodiment 44 to embodiment 51, wherein the endoscope assembly further comprises a control unit configured to analyze a force detected by the sensing unit.

Embodiment 53. The method according to any one of embodiment 44 to embodiment 52, wherein the adjusting of the endoscope assembly comprises: analyzing the force detected by the sensing unit; transmitting a signal to an alarm unit when the force exceeds a threshold force; and responding to an alarm generated by the alarm unit.

Embodiment 54. The method according to any one of embodiment 44 to embodiment 53, wherein the responding to the alarm generated by the alarm unit comprises stopping a movement of the overtube within the ureter or the access sheath.

Embodiment 55. The method according to any one of embodiment 44 to embodiment 54, wherein the responding to the alarm generated by the alarm unit comprises changing a movement direction of the overtube within the ureter or the access sheath.

Embodiment 56. The method according to any one of embodiment 44 to embodiment 55, wherein the threshold force is a predetermined force value, and wherein the control unit is configured to compare the measured force detected by the sensing unit with the predetermined force value.

Embodiment 57. The method according to any one of embodiment 44 to embodiment 56, further comprising providing a moving unit, wherein the endoscope assembly is operatively coupled to the moving unit such that the endoscope assembly is capable of being linearly moved along the moving unit.

Embodiment 58. The method according to any one of embodiment 44 to embodiment 57, wherein the moving unit is configured to be tilted with respect to a human body such that an angle of the endoscope assembly with respect to the patient is capable of being adjusted, and wherein the moving unit is capable of being moved vertically with respect to the human body, thereby being capable of adjusting a height of the endoscope assembly with respect to the patient.

Embodiment 59. The method according to any one of embodiment 44 to embodiment 58, wherein the operating of the endoscope assembly comprises: adjusting the height of the endoscope assembly by relatively adjusting a position of the moving human body vertically; and adjusting the angle of the endoscope assembly by adjusting an angle of the moving unit with respect to the human body.

### <ADDITIONAL EMBODIMENTS #2-1>

Embodiment 1. An endoscope driving assembly comprising:
a coupler for accommodating an endoscope; and
a mount sized and shaped to receive the coupler, the mount being configured to control the endoscope in response to a signal received from a master device,
wherein the coupler and the mount are configured such that the coupler and the mount are coupled so that the coupler and the mount are capable of being attached thereto and detached therefrom.

Embodiment 2. The endoscope driving assembly according to the preceding embodiment, wherein the endoscope driving assembly is capable of being moved along a driving body in response to a signal received from the master device.

Embodiment 3. The endoscope driving assembly according to any one of the preceding embodiments, wherein the endoscope driving assembly is capable of being moved in a plurality of directions along the driving body in response to a signal received from the master device.

Embodiment 4. The endoscope driving assembly according to any one of the preceding embodiments, wherein the mount is sized and shaped to accommodate another coupler and another endoscope having different sizes or different shapes from the endoscope.

Embodiment 5. The endoscope driving assembly according to any one of the preceding embodiments, wherein the coupler comprises: a coupler body accommodating at least a portion of the endoscope; and a main hole for accommodating a handle of the endoscope.

Embodiment 6. The endoscope driving assembly according to any one of the preceding embodiments, wherein the mount comprises: a mount body; a rotor configured to be rotated in a clockwise direction or a counter-clockwise direction in response to a signal received from the master device; and a handle holder coupled to the rotor and configured to rotate the handle of the endoscope when the endoscope is positioned within the coupler, wherein the coupler body is disposed on the mount body during assembly.

Embodiment 7. The endoscope driving assembly according to any one of the preceding embodiments, wherein the handle holder is replaceable.

Embodiment 8. The endoscope driving assembly according to any one of the preceding embodiments, further comprising a first detection target, wherein the first detection target is configured to detect a situation in which the endoscope is positioned within the coupler body.

Embodiment 9. The endoscope driving assembly according to any one of the preceding embodiments, further comprising a second detection target, wherein the second detection target is configured to detect a situation in which the coupler body is coupled to the mount body.

Embodiment 10. The endoscope driving assembly according to any one of the preceding embodiments, wherein the mount further comprises: a vertical extension part formed around the rotor in a circumferential direction; and a plurality of guide grooves formed on a surface of the mount body, wherein the vertical extension part extends in an upward direction from the surface of the mount body and surrounds the rotor, and wherein the plurality of guide grooves is formed on a surface of the vertical extension part.

Embodiment 11. The endoscope driving assembly according to any one of the preceding embodiments, wherein the coupler further comprises a plurality of guide protrusions configured to be accommodated in the plurality of guide grooves, wherein, when the plurality of guide protrusions is disposed within the plurality of guide grooves, the plurality of guide protrusions is configured to be rotated around a rotation axis of the rotor so that the coupler is fastened to the mount.

Embodiment 12. The endoscope driving assembly according to any one of the preceding embodiments, further comprising a hook, wherein the hook comprises: a hook body supported by the mount body; and a hook head that protrudes toward the coupler body from the hook body.

Embodiment 13. The endoscope driving assembly according to any one of the preceding embodiments, wherein the coupler further comprises an elastic protrusion, and wherein, when the coupler and the mount are coupled to each other, the elastic protrusion is in contact with the hook body.

Embodiment 14. Use of the endoscope assembly according to any one of the preceding embodiments for performing a surgical operation.

Embodiment 15. An endoscope assembly system comprising:
a master device provided with a screen and a controller; an endoscope driving assembly configured to accommodate an endoscope and to receive a signal from the master device; and a driving body accommodating the endoscope driving assembly, wherein the endoscope driving assembly is configured to be moved along the driving body in response to the signal from the master device.

Embodiment 16. The endoscope assembly system according to embodiment 15, wherein the master device is formed integrally with the endoscope driving assembly.

Embodiment 17. The endoscope assembly system according to embodiment 15 or embodiment 16, wherein the endoscope driving assembly comprises: a coupler accommodating the endoscope; and a mount sized and shaped to accommodate the coupler, and wherein the coupler and the mount are configured such that the coupler and the mount are coupled so that the coupler and the mount are capable of being attached thereto and detached therefrom.

Embodiment 18. The endoscope assembly system according to any one of embodiment 15 to embodiment 17, wherein the coupler comprises:
a coupler body accommodating at least a portion of the endoscope; and a main hole for accommodating a handle of the endoscope.

Embodiment 19. The endoscope assembly system according to any one of embodiment 15 to embodiment 18, wherein the mount comprises:
a mount body; a rotor configured to be rotated in a clockwise direction or a counter-clockwise direction in response to a signal received from the master device; a handle holder coupled to the rotor and configured to rotate the handle of the endoscope when the endoscope is positioned within the coupler, wherein the coupler body is disposed on the mount body during assembly.

Embodiment 20. The endoscope assembly system according to any one of embodiment 15 to embodiment 19, wherein the handle holder is replaceable.

Embodiment 21. The endoscope assembly system according to any one of embodiment 15 to embodiment 20, further comprising a first detection target, wherein the first detection target is configured to detect a situation in which the endoscope is positioned within the coupler body.

Embodiment 22. The endoscope assembly system according to any one of embodiment 15 to embodiment 21, further comprising a second detection target, wherein the second detection target is configured to detect a situation in which the coupler body is coupled to the mount body.

Embodiment 23. The endoscope assembly system according to any one of embodiment 15 to embodiment 22, wherein the mount further comprises:
a vertical extension part formed around the rotor in a circumferential direction; and a plurality of guide grooves formed on a surface of the mount body, wherein the vertical extension part extends in an upward direction from the surface of the mount body and surrounds the rotor, and wherein the plurality of guide grooves is formed on a surface of the vertical extension part.

Embodiment 24. The endoscope assembly system according to any one of embodiment 15 to embodiment 23, wherein the coupler further comprises a plurality of guide protrusions configured to be accommodated in the plurality of guide grooves, wherein, when the plurality of guide protrusions is disposed within the plurality of guide grooves, the plurality of guide protrusions is configured to be rotated around a rotation axis of the rotor so that the coupler is fastened to the mount.

Embodiment 25. The endoscope assembly system according to any one of embodiment 15 to embodiment 24, further comprising a hook,
wherein the hook comprises: a hook body supported by the mount body; and a hook head that protrudes toward the coupler body from the hook body.

Embodiment 26. The endoscope assembly system according to any one of embodiment 15 to embodiment 25, wherein the coupler further comprises an elastic protrusion, and wherein, when the coupler and the mount are coupled to each other, the elastic protrusion is in contact with the hook body.

Embodiment 27. The endoscope assembly system according to any one of embodiment 15 to embodiment 26, wherein the driving body comprises: a tube holder disposed on a surface of the driving body; a railing configured to accommodate the endoscope driving assembly; and an access sheath coupled to an end of the driving body, and wherein the access sheath is configured to be inserted into a patient's body during a surgical operation.

Embodiment 28. The endoscope assembly system according to any one of embodiment 15 to embodiment 27, wherein the access sheath is configured to accommodate a tube of the endoscope.

Embodiment 29. The endoscope assembly system according to any one of embodiment 15 to embodiment 27, wherein, during use the endoscope assembly system, the endoscope driving assembly is moved along the railing of the driving body.

Embodiment 30. Use of the endoscope assembly system according to any one of embodiment 15 to embodiment 29 for performing a surgical operation.

Embodiment 31. A method of operating an endoscope driving assembly, the method comprising: positioning an endoscope within a coupler; coupling the coupler and a mount to each other before or after the positioning; and driving the endoscope driving assembly by a signal for performing an operation.

Embodiment 32. The method according to embodiment 31, wherein the coupler comprises: a coupler body accommodating at least a portion of an endoscope; and a main hole for accommodating a handle of the endoscope, wherein the mount comprises: a mount body; a rotor configured to be rotated in a clockwise direction or a counter-clockwise direction in response to a signal received from a master device; and a handle holder coupled to the rotor and configured to rotate the handle of the endoscope when the endoscope is positioned within the coupler, and wherein the coupler body is disposed on the mount body during assembly.

Embodiment 33. The method according to embodiment 31 or embodiment 32, wherein the endoscope driving assembly further comprises a first detection target, and wherein the first detection target is configured to detect a situation in which the endoscope is positioned within the coupler body.

Embodiment 34. The method according to any one of embodiment 31 to embodiment 33, wherein the endoscope driving assembly further comprises a second detection target, and wherein the second detection target is configured to detect a situation in which the coupler body is coupled to the mount body.

Embodiment 35. The method according to any one of embodiment 31 to embodiment 34, wherein the mount further comprises: a vertical extension part formed around the rotor in a circumferential direction; and a plurality of guide grooves formed on a surface of the mount body, and wherein the coupler comprises a plurality of guide protrusions.

Embodiment 36. The method according to any one of embodiment 31 to embodiment 35, wherein the coupling of the coupler and the mount comprises: positioning the coupler body on the mount body such that the plurality of guide protrusions is disposed within the plurality of guide grooves; and fixing the coupler to the mount by rotating the plurality of guide protrusions around a rotation axis of the rotor.

Embodiment 37. The method according to any one of embodiment 31 to embodiment 36, wherein the mount further comprises a hook for engaging with the coupler when the coupler and the mount are coupled to each other, and wherein the coupler further comprises an elastic protrusion.

Embodiment 38. The method according to any one of embodiment 31 to embodiment 37, wherein the coupling of the coupler and the mount comprises positioning the coupler body on the mount body such that the elastic protrusion is engaged with the hook.

Embodiment 39. The method according to any one of embodiment 31 to embodiment 38, wherein the driving of the endoscope driving assembly comprises: inputting an operation into the master device; and receiving a signal from the master device, the signal causing an adjustment of the endoscope driving assembly.

Embodiment 40. The method according to any one of embodiment 31 to embodiment 39, wherein the rotor is configured to be rotated in the clockwise direction or the counter-clockwise direction in response to a signal received from the master device.

### <ADDITIONAL EMBODIMENTS #1-1>

Embodiment 1. A surgical robot assembly comprising:
an access sheath configured to be inserted into a subject;
an endoscope comprising an overtube configured to be inserted into the access sheath;
a surgical tool configured to be inserted into the overtube; and
a sensing unit for detecting a presence, absence or amount of a force applied to the access sheath.

Embodiment 2. The surgical robot assembly according to any one of the preceding embodiments, wherein the overtube is coupled to an end of the endoscope.

Embodiment 3. The surgical robot assembly according to any one of the preceding embodiments wherein the subject is human.

Embodiment 4. The surgical robot assembly according to any one of the preceding embodiments wherein the subject is an animal.

Embodiment 5. The surgical robot assembly according to any one of the preceding embodiments wherein the presence, absence, or amount of force is detected.

Embodiment 6. The surgical robot assembly according to any one of the preceding embodiments wherein the endoscope enters and/or exits the subject.

Embodiment 7. The surgical robot assembly according to any one of the preceding embodiments wherein a stone is removed from the subject by the surgical robot.

Embodiment 8. The surgical robot assembly according to any one of the preceding embodiments, wherein the access sheath comprises a first end and a second end opposite the first end, and
wherein the access sheath comprises an inlet at first end.

Embodiment 9. The surgical robot assembly according to any one of the preceding embodiments, wherein the inlet is cone-shaped.

Embodiment 10. The surgical robot assembly according to any one of the preceding embodiments, further comprising a support holder, and
wherein the support holder is operatively coupled to the inlet.

Embodiment 11. The surgical robot assembly according to any one of the preceding embodiments, wherein the support holder comprises a holding member and an arm member,
wherein the holding member is configured to couple to the inlet of the access sheath, and
wherein the arm is coupled to the holding member at a first end and a coupled to the sensing unit at a second end.

Embodiment 12. The surgical robot assembly according to any one of the preceding embodiments, wherein the arm member includes a curved portion, and
wherein the arm member is configured to act as a hinge point.

Embodiment 13. The surgical robot assembly according to any one of the preceding embodiments, wherein the overtube is movable within the access sheath in a first direction and a second direction opposite the first direction,
wherein, when the overtube is moved within the access sheath in the first direction, a first force is generated, and
wherein, when the overtube is moved within the access sheath in the second direction, a second force is generated.

Embodiment 14. The surgical robot assembly according to any one of the preceding embodiments, wherein the sensing unit measures each of the first force and the second force.

Embodiment 15. The surgical robot assembly according to any one of the preceding embodiments, wherein, when the overtube is moved in the first direction or the second direction, the first force or the second force is transmitted to the arm member.

Embodiment 16. The surgical robot assembly according to any one of the preceding embodiments, further comprising a guide tube, a wire and a basket disposed at an end of the wire,
wherein the wire is configured to be inserted through the guide tube, and
wherein the guide tube is configured tube is configured to be inserted through the access sheath.

Embodiment 17. The surgical robot assembly according to any one of the preceding embodiments, further comprising a control unit for analyzing the force detected by the sensing unit, and
wherein, when the force exceeds a threshold, the control unit operates an alarm unit.

Embodiment 18. The surgical robot assembly according to any one of the preceding embodiments, wherein the threshold is a predetermined force value.

Embodiment 19. The surgical robot assembly according to any one of the preceding embodiments, wherein the control unit compares the measured force detected by the sensing unit and to the predetermined force value.

Embodiment 20. The surgical robot assembly according to any one of the preceding embodiments, wherein the endoscope is configured to be coupled with a moving unit, and
wherein, during use, the endoscope is movable along the moving unit.

Embodiment 21. Use of the surgical robot assembly according to any one of the preceding embodiments for performing a surgical operation.

Embodiment 22. A surgical robot system comprising:
a master device having a screen and a controller;
a drive body;
a moving unit coupled to the drive body; and
a surgical robot assembly comprising an endoscope and configured to receive a signal from the master unit,
wherein in response to a signal from the master device, the surgical robot assembly moves relative the body via the moving unit.

Embodiment 23. The surgical robot system according to embodiment 22, wherein the surgical robot assembly further comprises:
an access sheath configured to be inserted into a subject;
an overtube configured to be inserted into the access sheath;
a surgical tool configured to be inserted into the overtube; and
a sensing unit for detecting a presence, absence or amount of a force applied to the access sheath.

Embodiment 24. The surgical robot system according to embodiment 22 or 23, wherein the overtube is coupled to an end of the endoscope.

Embodiment 25. The surgical robot system according to any one of embodiments 22-24, wherein the subject is human.

Embodiment 26. The surgical robot system according to any one of embodiments 22-24, wherein the subject is an animal.

Embodiment 27. The surgical robot system according to any one of embodiments 22-26, wherein the presence, absence, or amount of force is detected.

Embodiment 28. The surgical robot system according to any one of embodiments 22-27, wherein the endoscope enters and/or exits the subject.

Embodiment 29. The surgical robot system according to any one of embodiments 22-28, wherein a stone is removed from the subject by the surgical robot.

Embodiment 30. The surgical robot system according to any one of embodiments 22-29, wherein the access sheath comprises a first end and a second end opposite the first end, and
wherein the access sheath comprises an inlet at first end.

Embodiment 31. The surgical robot system according to any one of embodiments 22-30, wherein the inlet is cone-shaped.

Embodiment 32. The surgical robot system according to any one of embodiments 22-31, further comprising a support holder, and
wherein the support holder is operatively coupled to the inlet.

Embodiment 33. The surgical robot system according to any one of embodiments 22-32, wherein the support holder comprises a holding member and an arm member,
wherein the holding member is configured to couple to the inlet of the access sheath, and
wherein the arm is coupled to the holding member at a first end and a coupled to the sensing unit at a second end.

Embodiment 34. The surgical robot system according to any one of embodiments 22-33, wherein the arm member includes a curved portion, and
wherein the arm member is configured to act as a hinge point.

Embodiment 35. The surgical robot system according to any one of embodiments 22-34,
wherein the overtube is movable within the access sheath in a first direction and a second direction opposite the first direction,
wherein, when the overtube is moved within the access sheath in the first direction, a first force is generated, and
wherein, when the overtube is moved within the access sheath in the second direction, a second force is generated.

Embodiment 36. The surgical robot system according to any one of embodiments 22-35, wherein the sensing unit measures each of the first force and the second force.

Embodiment 37. The surgical robot system according to any one of embodiments 22-36, wherein, when the overtube is moved in the first direction or the second direction, the first force or the second force is transmitted to the arm member.

Embodiment 38. The surgical robot system according to any one of embodiments 22-37, further comprising a guide tube, a wire and a basket disposed at an end of the wire,
wherein the wire is configured to be inserted through the guide tube, and
wherein the guide tube is configured tube is configured to be inserted through the access sheath.

Embodiment 39. The surgical robot system according to any one of embodiments 22-38, further comprising a control unit for analyzing the force detected by the sensing unit, and
wherein, when the force exceeds a threshold, the control unit operates an alarm unit.

Embodiment 40. The surgical robot system according to any one of embodiments 22-39, wherein the threshold is a predetermined force value.

Embodiment 41. The surgical robot system according to any one of embodiments 22-40,
wherein the control unit compares the measured force detected by the sensing unit and to the predetermined force value.

Embodiment 42. The surgical robot system according to any one of embodiments 22-41, wherein the endoscope is configured to be coupled with a moving unit, and
wherein, during use, the endoscope is movable along the moving unit.

Embodiment 43. Use of the surgical robot system according to any one of embodiments 22-42 for performing a surgical operation.

Embodiment 44. A method of operating a surgical robot, the method comprising:
operating an endoscope assembly comprising an overtube, a surgical tool, an access sheath, and a sensing unit, and
adjusting the endoscope in response to signals received from the sensing device.

Embodiment 45. The method according to embodiment 44, wherein the access sheath comprises a first end and a second end opposite the first end, and
wherein the access sheath comprises an inlet at first end.

Embodiment 46. The method according to embodiment 44 or 45, wherein the endoscope assembly further comprises a support holder operatively coupled to the inlet, the support holder having a holding member and an arm member,
wherein the holding member is configured to couple to the inlet of the access sheath, and
wherein the arm is coupled to the holding member at a first end and a coupled to the sensing unit at a second end.

Embodiment 47. The method of any one of embodiments 44-46, wherein operating the endoscope assembly comprises:
inserting the access sheath into a patient,
inserting the overtube through the access sheath,
wherein, when the overtube is inserted through the access sheath in a first direction, a first force is generated, and
wherein the sensing unit detects and measures the first force.

Embodiment 48. The method of any one of embodiments 44-47, wherein, when the overtube is moved within the access sheath in a second direction, a second force is generated, and
wherein the sensing unit detects and measures the first force.

Embodiment 49. The method of any one of embodiments 44-48, wherein, when operating the endoscope assembly, the first force or the second force is transmitted to the arm member.

Embodiment 50. The method of any one of embodiments 44-49, wherein the endoscope further comprises a guide tube, a wire, and a basket disposed at an end of the wire.

Embodiment 51. The method of any one of embodiments 44-50, wherein operating the endoscope comprises:
inserting the guide tube through the access sheath,
inserting the wire and the basket through the guide tube.

Embodiment 52. The method of any one of embodiments 44-51, wherein the endoscope assembly further comprises a control unit for analyzing the force detected by the sensing unit.

Embodiment 53. The method of any one of embodiments 44-52, wherein adjusting the endoscope assembly comprises:
analyzing the force detected by the sensing unit,
transmitting a signal to an alarm unit when the force exceeds a threshold force,
responding to an alarm produced by the alarm unit.

Embodiment 54. The method of any one of embodiments 44-53, wherein responding to the alarm unit comprises ceasing movement of the overtube within the access sheath.

Embodiment 55. The method of any one of embodiments 44-54, wherein responding to the alarm unit comprises altering a direction of movement of the overtube within the access sheath.

Embodiment 56. The method of any one of embodiments 44-55, wherein the threshold force is a predetermined force value, and
wherein the control unit compares the measured force detected by the sensing unit and to the predetermined force value.

Embodiment 57. The method of any one of embodiments 44-56, further comprising providing a moving unit, and
wherein the endoscope assembly is operatively coupled to the moving unit, such that the endoscope assembly can be moved linearly along the moving unit.

Embodiment 58. The method of any one of embodiments 44-57, wherein the moving unit is configured to tilt relative a body such that an angle of the endoscope assembly relative the patient can be adjusted, and
wherein the moving unit can be moved vertically with respect to the body thereby adjusting a height of the endoscope relative to the patient.

Embodiment 59. The method of any one of embodiments 44-58, wherein operating the endoscope comprises:
adjusting the height of the endoscope assembly by adjusting the position of the moving relative the body in a vertical direction, and
adjusting the angle of the endoscope assembly by adjusting the angle of the moving unit relative the body.

### <FURTHER EMBODIMENTS #1-2>

Embodiment 1. A surgical robot assembly comprising:
an endoscope comprising an overtube configured to be inserted into a ureter of a subject;
a surgical tool configured to be inserted into the overtube; and
a sensing unit for detecting a presence, absence or amount of a force applied to the ureter.

Embodiment 2. The surgical robot assembly according to any one of the preceding embodiments, wherein the overtube is coupled to an end of the endoscope.

Embodiment 3. The surgical robot assembly according to any one of the preceding embodiments wherein the subject is human.

Embodiment 4. The surgical robot assembly according to any one of the preceding embodiments wherein the subject is an animal.

Embodiment 5. The surgical robot assembly according to any one of the preceding embodiments wherein the presence, absence, or amount of force is detected.

Embodiment 6. The surgical robot assembly according to any one of the preceding embodiments wherein the endoscope enters and/or exits the subject.

Embodiment 7. The surgical robot assembly according to any one of the preceding embodiments wherein a stone is removed from the subject by the surgical robot.

Embodiment 8. The surgical robot assembly according to any one of the preceding embodiments, further comprising an access sheath configured to be inserted into the subject, wherein the overtube is configured to be inserted into the access sheath, wherein the access sheath comprises a first end and a second end opposite the first end, and
wherein the access sheath comprises an inlet at first end.

Embodiment 9. The surgical robot assembly according to any one of the preceding embodiments, wherein the inlet is cone-shaped.

Embodiment 10. The surgical robot assembly according to any one of the preceding embodiments, further comprising a support holder, and
wherein the support holder is operatively coupled to the inlet.

Embodiment 11. The surgical robot assembly according to any one of the preceding embodiments, wherein the support holder comprises a holding member and an arm member,
wherein the holding member is configured to couple to the inlet of the ureter or the access sheath, and
wherein the arm is coupled to the holding member at a first end and a coupled to the sensing unit at a second end.

Embodiment 12. The surgical robot assembly according to any one of the preceding embodiments, wherein the arm member includes a curved portion, and
wherein the arm member is configured to act as a hinge point.

Embodiment 13. The surgical robot assembly according to any one of the preceding embodiments, wherein the overtube is movable within the ureter or the access sheath in a first direction and a second direction opposite the first direction,
wherein, when the overtube is moved within the ureter or the access sheath in the first direction, a first force is generated, and
wherein, when the overtube is moved within the ureter or the access sheath in the second direction, a second force is generated.

Embodiment 14. The surgical robot assembly according to any one of the preceding embodiments, wherein the sensing unit measures each of the first force and the second force.

Embodiment 15. The surgical robot assembly according to any one of the preceding embodiments, wherein, when the overtube is moved in the first direction or the second direction, the first force or the second force is transmitted to the arm member.

Embodiment 16. The surgical robot assembly according to any one of the preceding embodiments, further comprising a guide tube, a wire and a basket disposed at an end of the wire,
wherein the wire is configured to be inserted through the guide tube, and
wherein the guide tube is configured tube is configured to be inserted through the ureter or the access sheath.

Embodiment 17. The surgical robot assembly according to any one of the preceding embodiments, further comprising a control unit for analyzing the force detected by the sensing unit, and
wherein, when the force exceeds a threshold, the control unit operates an alarm unit.

Embodiment 18. The surgical robot assembly according to any one of the preceding embodiments, wherein the threshold is a predetermined force value.

Embodiment 19. The surgical robot assembly according to any one of the preceding embodiments, wherein the control unit compares the measured force detected by the sensing unit and to the predetermined force value.

Embodiment 20. The surgical robot assembly according to any one of the preceding embodiments, wherein the endoscope is configured to be coupled with a moving unit, and
wherein, during use, the endoscope is movable along the moving unit.

Embodiment 21. Use of the surgical robot assembly according to any one of the preceding embodiments for performing a surgical operation.

Embodiment 22. A surgical robot system comprising:
a master device having a screen and a controller;
a drive body;
a moving unit coupled to the drive body; and
a surgical robot assembly comprising an endoscope and configured to receive a signal from the master unit,
wherein in response to a signal from the master device, the surgical robot assembly moves relative the body via the moving unit.

Embodiment 23. The surgical robot system according to embodiment 22, wherein the surgical robot assembly further comprises:
an overtube configured to be inserted into a ureter of a subject;
a surgical tool configured to be inserted into the overtube; and
a sensing unit for detecting a presence, absence or amount of a force applied to the ureter.

Embodiment 24. The surgical robot system according to embodiment 22 or 23, wherein the overtube is coupled to an end of the endoscope.

Embodiment 25. The surgical robot system according to any one of embodiments 22-24, wherein the subject is human.

Embodiment 26. The surgical robot system according to any one of embodiments 22-24, wherein the subject is an animal.

Embodiment 27. The surgical robot system according to any one of embodiments 22-26, wherein the presence, absence, or amount of force is detected.

Embodiment 28. The surgical robot system according to any one of embodiments 22-27, wherein the endoscope enters and/or exits the subject.

Embodiment 29. The surgical robot system according to any one of embodiments 22-28, wherein a stone is removed from the subject by the surgical robot.

Embodiment 30. The surgical robot system according to any one of embodiments 22-29, further comprising an access sheath configured to be inserted into the subject, wherein the overtube is configured to be inserted into the access sheath, wherein the access sheath comprises a first end and a second end opposite the first end, and
wherein the access sheath comprises an inlet at first end.

Embodiment 31. The surgical robot system according to any one of embodiments 22-30, wherein the inlet is cone-shaped.

Embodiment 32. The surgical robot system according to any one of embodiments 22-31, further comprising a support holder, and
wherein the support holder is operatively coupled to the inlet.

Embodiment 33. The surgical robot system according to any one of embodiments 22-32, wherein the support holder comprises a holding member and an arm member,
wherein the holding member is configured to couple to the inlet of the ureter or the access sheath, and
wherein the arm is coupled to the holding member at a first end and a coupled to the sensing unit at a second end.

Embodiment 34. The surgical robot system according to any one of embodiments 22-33, wherein the arm member includes a curved portion, and
wherein the arm member is configured to act as a hinge point.

Embodiment 35. The surgical robot system according to any one of embodiments 22-34,
wherein the overtube is movable within the ureter or the access sheath in a first direction and a second direction opposite the first direction,
wherein, when the overtube is moved within the ureter or the access sheath in the first direction, a first force is generated, and
wherein, when the overtube is moved within the ureter or the access sheath in the second direction, a second force is generated.

Embodiment 36. The surgical robot system according to any one of embodiments 22-35, wherein the sensing unit measures each of the first force and the second force.

Embodiment 37. The surgical robot system according to any one of embodiments 22-36, wherein, when the overtube is moved in the first direction or the second direction, the first force or the second force is transmitted to the arm member.

Embodiment 38. The surgical robot system according to any one of embodiments 22-37, further comprising a guide tube, a wire and a basket disposed at an end of the wire,
wherein the wire is configured to be inserted through the guide tube, and
wherein the guide tube is configured tube is configured to be inserted through the ureter or the access sheath.

Embodiment 39. The surgical robot system according to any one of embodiments 22-38, further comprising a control unit for analyzing the force detected by the sensing unit, and
wherein, when the force exceeds a threshold, the control unit operates an alarm unit.

Embodiment 40. The surgical robot system according to any one of embodiments 22-39, wherein the threshold is a predetermined force value.

Embodiment 41. The surgical robot system according to any one of embodiments 22-40,
wherein the control unit compares the measured force detected by the sensing unit and to the predetermined force value.

Embodiment 42. The surgical robot system according to any one of embodiments 22-41, wherein the endoscope is configured to be coupled with a moving unit, and
wherein, during use, the endoscope is movable along the moving unit.

Embodiment 43. Use of the surgical robot system according to any one of embodiments 22-42 for performing a surgical operation.

Embodiment 44. A method of operating a surgical robot, the method comprising:
operating an endoscope assembly comprising an overtube, a surgical tool, and a sensing unit, and
adjusting the endoscope in response to signals received from the sensing device.

Embodiment 45. The method according to embodiment 44, wherein the endoscope assembly further comprises an access sheath,
wherein the access sheath comprises a first end and a second end opposite the first end, and
wherein the access sheath comprises an inlet at first end.

Embodiment 46. The method according to embodiment 44 or 45, wherein the endoscope assembly further comprises a support holder operatively coupled to the inlet, the support holder having a holding member and an arm member,
wherein the holding member is configured to couple to the inlet of the ureter or the access sheath, and
wherein the arm is coupled to the holding member at a first end and a coupled to the sensing unit at a second end.

Embodiment 47. The method of any one of embodiments 44-46, wherein operating the endoscope assembly comprises:
optionally inserting the access sheath into a patient,
inserting the overtube through the ureter or the access sheath,
wherein, when the overtube is inserted through the ureter or the access sheath in a first direction, a first force is generated, and
wherein the sensing unit detects and measures the first force.

Embodiment 48. The method of any one of embodiments 44-47, wherein, when the overtube is moved within the ureter or the access sheath in a second direction, a second force is generated, and
wherein the sensing unit detects and measures the first force.

Embodiment 49. The method of any one of embodiments 44-48, wherein, when operating the endoscope assembly, the first force or the second force is transmitted to the arm member.

Embodiment 50. The method of any one of embodiments 44-49, wherein the endoscope further comprises a guide tube, a wire, and a basket disposed at an end of the wire.

Embodiment 51. The method of any one of embodiments 44-50, wherein operating the endoscope comprises:
inserting the guide tube through the ureter or the access sheath,
inserting the wire and the basket through the guide tube.

Embodiment 52. The method of any one of embodiments 44-51, wherein the endoscope assembly further comprises a control unit for analyzing the force detected by the sensing unit.

Embodiment 53. The method of any one of embodiments 44-52, wherein adjusting the endoscope assembly comprises:
analyzing the force detected by the sensing unit,
transmitting a signal to an alarm unit when the force exceeds a threshold force,
responding to an alarm produced by the alarm unit.

Embodiment 54. The method of any one of embodiments 44-53, wherein responding to the alarm unit comprises ceasing movement of the overtube within the ureter or the access sheath.

Embodiment 55. The method of any one of embodiments 44-54, wherein responding to the alarm unit comprises altering a direction of movement of the overtube within the ureter or the access sheath.

Embodiment 56. The method of any one of embodiments 44-55, wherein the threshold force is a predetermined force value, and
wherein the control unit compares the measured force detected by the sensing unit and to the predetermined force value.

Embodiment 57. The method of any one of embodiments 44-56, further comprising providing a moving unit, and
wherein the endoscope assembly is operatively coupled to the moving unit, such that the endoscope assembly can be moved linearly along the moving unit.

Embodiment 58. The method of any one of embodiments 44-57, wherein the moving unit is configured to tilt relative a body such that an angle of the endoscope assembly relative the patient can be adjusted, and
wherein the moving unit can be moved vertically with respect to the body thereby adjusting a height of the endoscope relative to the patient.

Embodiment 59. The method of any one of embodiments 44-58, wherein operating the endoscope comprises:
adjusting the height of the endoscope assembly by adjusting the position of the moving relative the body in a vertical direction, and
adjusting the angle of the endoscope assembly by adjusting the angle of the moving unit relative the body.

### <ADDITIONAL EMBODIMENTS #2-1>

Embodiment 1. An endoscope drive assembly comprising:
a coupler for receiving an endoscope; and
a mount sized and shaped to receive the coupler, the mount controlling the endoscope in response to a signal received from the master device,
wherein the coupler and the mount are configured to be removably coupled.

Embodiment 2. The endoscope drive assembly according to any one of the preceding embodiments, wherein the wherein, the endoscope drive assembly is movable along a drive body in response to a signal received from a master device.

Embodiment 3. The endoscope drive assembly according to any one of the preceding embodiments, wherein the wherein, the endoscope drive assembly is movable along a drive body in a plurality of directions in response to a signal received from a master device.

Embodiment 4. The endoscope drive assembly according to any one of the preceding embodiments wherein the mount is sized and shaped to receive another coupler and another endoscope having a different size or shape from said endoscope.

Embodiment 5. The endoscope drive assembly according to any one of the preceding embodiments, wherein the coupler comprises:
a coupler body for receiving at least a portion of the endoscope; and
a main hole for receiving a handle of the endoscope.

Embodiment 6. The endoscope drive assembly according to any one of the preceding embodiments, wherein the mount comprises:
a mount body,
a rotor configured to rotate in a clockwise or counterclockwise position in response to a signal received from a master device; and
a handle holder coupled to the rotor and configured to rotate a handle of the endoscope when the endoscope is positioned within the coupler, and
wherein, when assembled, the coupler body is disposed on the mount body.

Embodiment 7. The endoscope drive assembly according to any one of the preceding embodiments, wherein the handle holder is replaceable.

Embodiment 8. The endoscope drive assembly according to any one of the preceding embodiments, further comprising a first detection target, and
wherein the first detection target is configured to detect when the endoscope is positioned within the coupler body.

Embodiment 9. The endoscope drive assembly according to any one of the preceding embodiments, further comprising a second detection target, and
wherein the second detection target is configured to detect when the coupler body is coupled to the mount body.

Embodiment 10. The endoscope drive assembly according to any one of the preceding embodiments, wherein the mount further comprises:
a vertical extension formed circumferentially around the rotor; and
a plurality of guide grooves formed on a surface of the mount body,
wherein the vertical extension extends in an upward direction from the surface of the mount body and surrounds the rotor, and
wherein the plurality of guide grooves are formed on a surface of the vertical extension.

Embodiment 11. The endoscope drive assembly according to any one of the preceding embodiments, wherein the coupler further comprises a plurality of guide protrusions configured to be received within the plurality of guide grooves,
wherein, when the plurality of guide protrusions are disposed within the plurality of guide grooves, the plurality of guide protrusions are configured to rotate about a rotational axis of the rotor to fasten the coupler to the mount.

Embodiment 12. The endoscope drive assembly according to any one of the preceding embodiments, wherein the hook comprises:
a hook body supported by the mount body; and
a hook head protruding from the hook body in a direction toward the coupler body.

Embodiment 13. The endoscope drive assembly according to any one of the preceding embodiments, wherein the coupler further comprises an elastic protrusion, and
wherein, when the coupler and the mount are coupled, the elastic protrusion contacts the hook body.

Embodiment 14. Use of the endoscope assembly device according to any one of the preceding embodiments for performing a surgical operation.

Embodiment 15. An endoscope assembly system comprising:
a master device having a screen and a controller;
an endoscope drive assembly for receiving an endoscope and configured to receive a signal from the master device; and
a drive body for receiving the endoscope drive assembly,
wherein, in response to the signal from the master device, the endoscope drive assembly moves along the drive body.

Embodiment 16. The endoscope assembly system according to embodiment 15, wherein the master device is integrally formed with the endoscope drive assembly.

Embodiment 17. The endoscope assembly system according to embodiment 15 or 16, wherein the endoscope drive assembly comprises:
a coupler for receiving the endoscope; and
a mount sized and shaped to receive the coupler,
wherein the coupler and the mount are configured to be removably coupled.

Embodiment 18. The endoscope assembly system according to any one of embodiments 15-17, wherein the coupler comprises:
a coupler body for receiving at least a portion of the endoscope; and
a main hole for receiving a handle of the endoscope.

Embodiment 19. The endoscope assembly system according to any one of embodiments 15-18, wherein the mount comprises:
a mount body,
a rotor configured to rotate in a clockwise or counterclockwise position in response to a signal received from a master device; and
a handle holder coupled to the rotor and configured to rotate a handle of the endoscope when the endoscope is positioned within the coupler, and
wherein, when assembled, the coupler body is disposed on the mount body.

Embodiment 20. The endoscope assembly system according to any one of embodiments 15-19, wherein the handle holder is replaceable.

Embodiment 21. The endoscope assembly system according to any one of embodiments 15-20, further comprising a first detection target, and
wherein the first detection target is configured to detect when the endoscope is positioned within the coupler body.

Embodiment 22. The endoscope assembly system according to any one of embodiments 15-21, further comprising a second detection target, and
wherein the second detection target is configured to detect when the coupler body is coupled to the mount body.

Embodiment 23. The endoscope assembly system according to any one of embodiments 15-22, wherein the mount further comprises:
a vertical extension formed circumferentially around the rotor; and
a plurality of guide grooves formed on a surface of the mount body,
wherein the vertical extension extends in an upward direction from the surface of the mount body and surrounds the rotor, and
wherein the plurality of guide grooves are formed on a surface of the vertical extension.

Embodiment 24. The endoscope assembly system according to any one of embodiments 15-23, wherein the coupler further comprises a plurality of guide protrusions configured to be received within the plurality of guide grooves,
wherein, when the plurality of guide protrusions are disposed within the plurality of guide grooves, the plurality of guide protrusions are configured to rotate about a rotational axis of the rotor to fasten the coupler to the mount.

Embodiment 25. The endoscope assembly system according to any one of embodiments 15-24, wherein the hook comprises:
a hook body supported by the mount body; and
a hook head protruding from the hook body in a direction toward the coupler body.

Embodiment 26. The endoscope assembly system according to any one of embodiments 15-25, wherein the coupler further comprises an elastic protrusion, and
wherein, when the coupler and the mount are coupled, the elastic protrusion contacts the hook body.

Embodiment 27. The endoscope assembly system according to any one of embodiments 15-26, wherein the drive body further comprises:
a tube holder disposed on a surface of the drive body;
a railing configured to receive the endoscope drive assembly; and
an access sheath coupled to an end of the drive body,
wherein the access sheath is configured to be inserted into a patient's body during surgery.

Embodiment 28. The endoscope assembly system according to any one of embodiments 15-27, wherein the access sheath is configured to receive a tube of an endoscope.

Embodiment 29. The endoscope assembly system according to any one of embodiments 15-28, wherein, in use, the endoscope drive assembly moves along the railing of the drive body.

Embodiment 30. Use of the endoscope assembly device system according to any one of embodiments 15-29 for performing a surgical operation.

Embodiment 31. A method of operating an endoscope drive assembly, the method comprising:
positioning an endoscope within the coupler,
coupling the coupler and the mount before or after the positioning, and
actuating the endoscope drive assembly via a signal for performing an operation.

Embodiment 32. The method according to embodiment 31, wherein the coupler comprises:
a coupler body for receiving at least a portion of the endoscope; and
a main hole for receiving a handle of the endoscope,
wherein the mount comprises:
   a mount body,
   a rotor configured to rotate in a clockwise or counterclockwise position in response to a signal received from a master device; and
   a handle holder coupled to the rotor and configured to rotate a handle of the endoscope when the endoscope is positioned within the coupler, and
   wherein, when assembled, the coupler body is disposed on the mount body.

Embodiment 33. The method according to embodiment 31 or 32, wherein the endoscope drive assembly further comprises a first detection target, and
wherein the first detection target is configured to detect when the endoscope is positioned within the coupler body.

Embodiment 34. The method according to any one of embodiments 31-33, wherein the endoscope drive assembly further comprises a second detection target, and
wherein the second detection target is configured to detect when the coupler body is coupled to the mount body.

Embodiment 35. The method according to any one of embodiments 31-34, wherein the mount further comprises:
a vertical extension formed circumferentially around the rotor; and
a plurality of guide grooves formed on a surface of the mount body, and
wherein the coupler further comprises a plurality of guide protrusions

Embodiment 36. The method according to any one of embodiments 31-35, wherein coupling the coupler and the mount comprises:
positioning the coupler body on the mount body such that the plurality of guide protrusions are disposed within the plurality of guide grooves, and
rotating rotate about a rotational axis of the rotor to fasten the coupler to the mount.

Embodiment 37. The method according to any one of embodiments 31-36, wherein the mount further comprises a hook for engaging with the coupler when the coupler and the mount are coupled, and
wherein the coupler further comprises an elastic protrusion.

Embodiment 38. The method according to any one of embodiments 31-37, wherein coupling the coupler and the mount comprises positioning the coupler body on the mount body such that the elastic protrusion engages the hook.

Embodiment 39. The method according to any one of embodiments 31-38, wherein actuating the endoscope drive assembly comprises:
inputting an operation on a master device,
receiving a signal from the master device that causes adjustment of the endoscope device assembly.

Embodiment 40. The method according to any one of embodiments 31-39, wherein, in response to receiving a signal from the master device, the rotor rotates in a clockwise or counterclockwise direction.

## Claims

1. A surgical robot comprising:
an endoscope having an overtube configured to be inserted into an inner portion of a human body;
a surgical tool configured to be inserted into an inner portion of the overtube; and
a force sensing unit configured to measure a force generated when the endoscope or the surgical tool is inserted into or withdrawn from the inner portion of the human body.

2. The surgical robot of claim 1, further comprising:
an access sheath configured to be inserted into the inner portion of the human body,
wherein the force sensing unit is configured to measure a force applied to a ureter when the access sheath is inserted into the ureter in the human body without inserting the endoscope or the surgical tool.

3. The surgical robot of claim 1, further comprising:
an access sheath configured to be inserted into the inner portion of the human body,
wherein the endoscope is inserted into an inner portion of the access sheath, and
wherein the force sensing unit is configured to measure a force applied to the access sheath when the endoscope or the surgical tool is inserted into or withdrawn from the access sheath for removing a stone formed in the human body.

4. The surgical robot of claim 1, further comprising:
an access sheath configured to be inserted into the inner portion of the human body;
a mounting unit on which the endoscope is mounted such that the endoscope is capable of being attached to and detached from the mounting unit; and
a moving unit configured such that the mounting unit is capable of being reciprocated,
wherein the moving unit comprises a support holder configured to hold the access sheath such that the access sheath is capable of being attached to and detached from the support holder.

5. The surgical robot of claim 4, wherein the support holder comprises:
a holding part configured to hold the access sheath; and
an arm part connected to the holding part and coupled to the moving unit,
wherein the force sensing unit is fixed to an inner portion of the moving unit, and
wherein the force sensing unit is configured to measure a force transmitted to the arm part by friction or jam generated during a process of inserting the overtube into the access sheath or withdrawing the overtube from the access sheath.

6. The surgical robot of claim 1, further comprising:
a moving unit provided with a support holder,
wherein the endoscope is mounted on the support holder, and
wherein a force acting on the endoscope is measured by the force sensing unit through the support holder.

7. The surgical robot of claim 1, further comprising:
an access sheath configured to be inserted into the inner portion of the human body; and
a control unit configured to analyze a force applied to the endoscope or the access sheath by the force sensing unit,
wherein the control unit is configured to stop an operation of the endoscope when a measured value measured by the force sensing unit exceeds a reference value.

8. The surgical robot of claim 1, further comprising:
an access sheath configured to be inserted into the inner portion of the human body;
a control unit configured to analyze a force applied to the endoscope or the access sheath by the force sensing unit; and
an alarm unit configured to be operated according to a measured value measured by the force sensing unit,
wherein, in a situation in which the measured value measured by the force sensing unit exceeds a reference value, the control unit is configured to operate the alarm unit so as to notify the situation.

9. The surgical robot of claim 1, further comprising:
an access sheath configured to be inserted into the inner portion of the human body; and
a moving unit provided with a support holder,
wherein the inner portion of the human body is a ureter,
wherein, after the access sheath is inserted into an inner portion of the ureter, the access sheath is coupled to the support holder such that the access sheath is capable of being attached to and detached from the support holder, and
wherein the force sensing unit is configured to measure a force applied to the access sheath by the ureter before the endoscope or the surgical tool is inserted into the access sheath.

10. The surgical robot of claim 1, further comprising:
an access sheath configured to be inserted into the inner portion of the human body; and
a moving unit provided with a support holder,
wherein the inner portion of the human body is a ureter,
wherein the access sheath is coupled to the support holder such that the access sheath is capable of being attached to and detached from the support holder, and
wherein, during performing a surgical operation by using the surgical tool, the force sensing unit is configured to measure a force applied to the access sheath by the ureter that is moved by a patient's breath.

11. The surgical robot of claim 1, further comprising:
an access sheath configured to be inserted into the inner portion of the human body;
a moving unit provided with a support holder configured to support the access sheath such that the access sheath is capable of being attached to and detached from the support holder; and
a control unit configured to analyze a force applied to the endoscope or the access sheath by the force sensing unit,
wherein the force sensing unit is provided on an end side of the support holder,
wherein the surgical robot further comprises an actuator configured to move the support holder and the force sensing unit, and
wherein, when a measured value measured by the force sensing unit exceeds a reference value, the control unit is configured to operate the actuator so that the support holder and the force sensing unit are moved from the moving unit so as to reduce a force applied to the access sheath.

12. A surgical robot comprising:
an endoscope having an overtube configured to be inserted into an inner portion of a human body;
a surgical tool configured to be inserted into an inner portion of the overtube;
an access sheath configured to be inserted into the inner portion of the human body; and
a force sensing unit configured to measure a force acting between the human body and the access sheath.

13. A surgical robot configured to support and drive an endoscopic device disposed in a slave device, the surgical robot comprising:
a coupler configured to support the endoscopic device; and
a mount on which the coupler is mounted and which is configured to control the endoscopic device by receiving a signal from a master device,
wherein the coupler is provided such that the coupler is capable of being replaced so that the coupler is capable of supporting a plurality of endoscopic devices having different shapes.

14. The surgical robot of claim 13, wherein the coupler comprises:
a coupler body capable of accommodating at least a portion of the endoscopic device; and
a main hole which is formed through the coupler body and in which a handle of the endoscopic device is accommodated.

15. The surgical robot of claim 14, wherein the mount comprises:
a mount body configured to be fastened to the coupler body;
a rotor disposed on the mount body and configured such that the rotor is capable of being rotated in any one direction by receiving a signal from the master device; and
a handle holder connected to the rotor and configured to rotate the handle of the endoscopic device while the handle of the endoscopic device is accommodated in the main hole of the coupler.

16. The surgical robot of claim 15, wherein the handle holder is provided such that the handle holder is capable of being replaced.

17. The surgical robot of claim 15, wherein the coupler further comprises:
a first detection target configured to be moved in a direction toward the mount body by being pressed by the endoscopic device while the mount body and the coupler body are fastened to each other.

18. The surgical robot of claim 17, wherein the coupler further comprises:
a second detection target disposed in a surface of the coupler body facing the mount body.

19. The surgical robot of claim 18, wherein the mount further comprises:
a first sensor configured to detect the first detection target that is moved in the direction toward the mount body; and
a second sensor configured to detect the second detection target while the coupler body is fastened to the mount body.

20. The surgical robot of claim 15, wherein the mount further comprises:
a vertical extension part formed along a circumference of the rotor around a rotation axis of the rotor; and
a plurality of guide grooves formed on an outer circumferential surface of the vertical extension part by being spaced apart from each other.

21. The surgical robot of claim 20, wherein the coupler further comprises:
a plurality of guide protrusions configured to be accommodated in the plurality of guide grooves, respectively,
wherein the coupler is configured to be fastened to the mount by being rotated in one direction around the rotation axis of the rotor while the guide protrusions are accommodated in the guide grooves.

22. The surgical robot of claim 21, wherein the mount further comprises:
a hook provided such that the hook is capable of being caught on the coupler while the mount is fastened to the coupler.

23. The surgical robot of claim 22, wherein the hook comprises:
a hook body supported by the mount body; and
a hook head that protrudes toward the coupler body from the hook body.

24. The surgical robot of claim 23, wherein the coupler further comprises:
an elastic protrusion disposed on a surface of the coupler body facing the hook,
wherein the elastic protrusion is configured to press the hook body while the coupler body is caught on the hook head.
